(19)

Europäisches Patentamt

European Patent Office

Office européen des brevets

(11) **EP 4 734 014 A1**

(12) **EUROPEAN PATENT APPLICATION**

(43) Date of publication:
**29.04.2026 Bulletin 2026/18**

(21) Application number: **24306810.3**

(22) Date of filing: **25.10.2024**

(51) International Patent Classification (IPC):
**G06N 10/60** (2022.01)     **G06N 10/20** (2022.01)
**G16C 60/00** (2019.01)

(52) Cooperative Patent Classification (CPC):
**G06N 10/20; G06N 10/60;** G16C 10/00;
G16C 60/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **BULL SAS
78340 Les Clayes-Sous-Bois (FR)**
• **Centre National de la Recherche Scientifique
75016 Paris (FR)**
• **Ecole Polytechnique
91120 Palaiseau (FR)**

(72) Inventors:
• **AYRAL, Thomas
78000 VERSAILLES (FR)**
• **BERTRAND, Corentin
78000 VERSAILLES (FR)**
• **BESSERVE, Pauline
69210 FLEURIEUX SUR L'ARBRESLE (FR)**
• **FERRERO, Michel
92330 SCEAUX (FR)**

(74) Representative: **Plasseraud IP
104 Rue de Richelieu
CS92104
75080 Paris Cedex 02 (FR)**

(54) **METHOD FOR DETERMINING A QUANTUM PHASE PARAMETER OF A SOLID-STATE MATERIAL SAMPLE AND APPARATUS FOR IMPLEMENTING THE SAME**

(57)     A computer-implemented method for determining a quantum phase parameter characterizing a property of a quantum phase of a solid-state material sample, which comprises determining, for a bath site of an impurity-bath model of the solid-state material sample, respective parameters $V_p$ and $\varepsilon_p$ of the bath site, where $V_p$ represents a coupling strength of the bath site, and $\varepsilon_p$ represents an energy of the bath site, configuring a quantum circuit for computing a Green's function of the solid-state material sample corresponding to the impurity-bath model, wherein one or more quantum gates of the quantum circuit are configured based on the determined respective parameters $V_p$ and $\varepsilon_p$, wherein the quantum circuit is configured to use one or more noisy qubits, wherein respective noise patterns of the noisy qubits are used to represent dissipation of the impurity-bath model; and executing, on a quantum computer, the quantum circuit to determine an estimate of the quantum phase parameter.

Determining, for a bath site of an impurity-bath model of the solid-state material sample comprising one or more impurity sites representing an impurity and one or more bath sites representing a bath, respective parameters $V_p$ and $\varepsilon_p$ of the bath site of index $p$, where $V_p$ represents a strength of a coupling between the bath site of index $p$ and the impurity, and $\varepsilon_p$ represents an energy of the bath site of index $p$  — 21

Configuring a quantum circuit for computing a Green's function of the solid-state material sample corresponding to the impurity-bath model, one or more quantum gates of the quantum circuit being configured based on the determined respective parameters $V_p$ and $\varepsilon_p$, the quantum circuit being configured to use one or more noisy qubits, and respective amplitude damping noise patterns of the noisy qubits being used to represent dissipation of the impurity-bath model — 22

Execute on a quantum computer the quantum circuit to determine an estimate of the quantum phase parameter — 23

20

**FIG. 7**

**Description**

The present subject disclosure relates to the field of quantum computing, in particular to the determination of a parameter characterizing a property of a solid-state material by solving an impurity problem of a sample of the solid-state material using a quantum computer, and a quantum computer configured for performing such determination.

Quantum Computing is a field of computer science designed to solve some advanced problems faster than a classical computer, by using quantum mechanics. In this context, the term "classical" is often used to refer to a computer, a computing environment, a computing device (e.g. a processor) or a computing field which, as opposed to a reference to the term "quantum", does not use quantum mechanics.

The emerging field of Quantum Computing will have a significant impact in High Performance Computing (HPC), as HPC is already reaching the limit of classical computing. Consequently, HPC centers that only use classical computing may evolve to integrate quantum devices such as one or more Quantum Processing Units (QPUs) in the near future, to prepare for this new quantum revolution.

Among various methods to determine the electronic structure of strongly correlated materials, Dynamical Mean Field Theory (DMFT) is one of the workhorses of strongly-correlated electron systems thanks to its ability to capture Fermi-liquid as well as Mott physics, a feature not shared by other simpler embeddings.

As a consequence, DMFT is widely used in material science, for example for new solid-state material design or investigations of properties of solid-state materials.

The bottleneck of the numerical resolution of Dynamical Mean Field Theory (DMFT) is the solution of the impurity-bath model, which becomes exponentially difficult with the number of correlated orbitals. Hybrid quantum classical methods have been proposed that set out to solve this problem on ever-improving quantum processors. Yet, all previous approaches neglect the fact that the number of requisite bath sites (uncorrelated orbitals) in DMFT is very large and is thus unsuitable for a computation on near-term quantum processors.

There is therefore a need for an improved method for solving an impurity problem of a solid-state material sample that addresses the drawbacks and shortcomings of the conventional technology in the art.

In particular, it is an object of the present subject disclosure to provide an improved method for solving an impurity problem of a solid-state material sample and apparatuses for implementing the same that address the drawbacks and shortcomings of the conventional technology in the art.

Another object of the present subject disclosure is to provide an improved method for determining a parameter characterizing a property of a solid-state material by solving an impurity problem of a sample of the solid-state material and apparatuses for implementing the same that address the drawbacks and shortcomings of the conventional technology in the art.

Yet another object of the present subject disclosure is to provide an improved method for designing a new solid-state material by solving an impurity problem and apparatuses for implementing the same that address the drawbacks and shortcomings of the conventional technology in the art.

Yet another object of the present subject disclosure is to provide an improved method for investigating a property of a solid-state material by solving an impurity problem and apparatuses for implementing the same that address the drawbacks and shortcomings of the conventional technology in the art.

To achieve these objects and other advantages and in accordance with the purpose of the present subject disclosure, as embodied and broadly described herein, in one aspect of the present subject disclosure, a computer-implemented method for determining a quantum phase parameter characterizing a property of a quantum phase of a solid-state material sample is proposed. In one or more embodiments, the proposed method comprises: Determining, for a bath site of an (dissipative) impurity-bath model of the solid-state material sample comprising one or more impurity sites representing an impurity and one or more bath sites representing a bath, respective parameters $V_p$ and $\varepsilon_p$ of the bath site of index $p$, where $V_p$ represents a strength of a coupling between the bath site of index p and the impurity, and $\varepsilon_p$ represents an energy of the bath site of index $p$; configuring a quantum circuit for computing a Green's function of the solid-state material sample corresponding to the impurity-bath model, wherein one or more quantum gates of the quantum circuit are configured based on the determined respective parameters $V_p$ and $\varepsilon_p$, wherein the quantum circuit is configured to use one or more noisy qubits, wherein respective (amplitude damping) noise patterns of the noisy qubits are used to represent dissipation of the impurity-bath model; and executing, on a quantum computer, the quantum circuit to determine an estimate of the quantum phase parameter.

The proposed method advantageously addresses the above-mentioned numerical resolution issue of DMFT (numerical resolution of the impurity problem) by using noise in a quantum computer to reduce the number of bath sites thanks to an improved (more economical) fitting of the DMFT hybridization function. This advantageously paves the way for a solution of DMFT equations with a mixed hybrid quantum-classical algorithm which is proposed in the present subject disclosure.

Embodiments of the proposed scheme may advantageously be used for investigating properties of any solid-state material, including a crystal, and for producing and using such solid-state material according to the properties determined thanks to the proposed scheme.

In one or more embodiments, the quantum circuit may be configured to operate on a number of qubits that corresponds to a number of sites of the bath of the impurity-bath problem plus the number of correlated sites of the impurity-bath problem.

In one or more embodiments, the proposed method may further comprise: generate one or more noisy qubits by adding noise to one or more respective non-noisy qubits, and using the generated noisy qubits for executing the quantum circuit.

In one or more embodiments, the quantum circuit may be configured to operate on a number of noisy qubits that corresponds to a number of sites of the bath of the impurity-bath problem.

In one or more embodiments, the proposed method may further comprise: producing the solid-state material sample for using the determined parameter characterizing the property of a quantum phase of the solid-state material sample.

In one or more embodiments, the quantum circuit may be executed on the quantum computer to compute an estimate of a Green's function of the solid-state material sample. In one or more embodiments, the estimate of the quantum phase parameter may be determined based on the computed Green's function.

In one or more embodiments, the proposed method may further comprise: performing one or more iterations of an impurity-bath model resolution loop for a bath site of index $p$. In one or more embodiments, an iteration of index $j$ of the impurity-bath model resolution loop may comprise one or more of the following acts: computing (102), based on a structure parameter $\varepsilon(k)$ (and, in some embodiments, an electronic interactions parameter $U$) of the solid-state material sample, and a parameter $\Sigma j(\omega)$, estimates of values of a local Green's function of the solid state material expressed as the following complex variable function: $G_{loc,j}(\omega) = \sum_k \frac{1}{\omega - \epsilon(k) - \Sigma_j(\omega)}$ ; based on the estimates of values of the Green's function of the solid state material $G_{loc,j}(\omega)$ computed for the iteration $j$, and values of the parameter $\Sigma_j(\omega)$, computing (103) estimates of values of a hybridization function, for example based on the following hybridization function: $\Delta_j(\omega) = \omega - G_{loc,j}^{-1}(\omega) - \Sigma_j(\omega)$ ; performing (104) a fitting operation to fit the hybridization function $\Delta_j(\omega)$ with a sum of Lorentzian functions, in order to determine a set of bath parameters $\varepsilon_{p,j}$ and $V_{p,j}$; computing (105), based on the set of bath parameters $\varepsilon_{p,j}$ and $V_{p,j}$, estimates of values of an impurity Green's function $G_{imp,j}(\omega)$ corresponding the impurity-bath model of the solid state material; and computing (106), based on the estimates of values of the impurity Green's function $G_{imp,j}(\omega)$, estimates of values of a parameter $\Sigma_j'(\omega)$ . In some embodiments, the sum of Lorentzian functions may be based on: $\sum_{p \in \{1,...,n\}} \frac{V_p^2}{\omega - \epsilon_p + i\eta}$ where $\varepsilon_p$ and $V_p$ are the bath parameters of the impurity-bath model, and $\eta$ is a (small, e.g. infinitesimal) real positive number. In some embodiments, the parameter $\Sigma_j(\omega)$ may be set to an initial value $\Sigma_0(\omega)$ for performing the first iteration of the impurity-bath model resolution loop.

In one or more embodiments, the quantum circuit may be configured for performing the computing (105), based on the set of bath parameters $\varepsilon_{p,j}$ and $V_{p,j}$, of the estimates of values of the impurity Green's function $G_{imp,j}(\omega)$ corresponding to the impurity-bath model of the solid state material, when executed on a noisy quantum computer.

In one or more embodiments, the quantum circuit for computing the Green's function of the solid-state material sample may be configured for performing a quantum computation with $m + l$ perfect (non-noisy qubits) qubits, and $n$ noisy qubits, where the number $m$ is a non-zero natural integer that is a number of impurity qubits corresponding to a number of sites in the impurity of the impurity-bath model, the number $l$ is a natural integer that is a number of additional qubits corresponding to a number of additional fermionic sites added to the impurity-bath model, and the number $n$ of noisy qubits is a non-zero natural integer that is a number of bath qubits corresponding to a number of bath fermionic sites of the impurity bath model.

In one or more embodiments, the proposed method may further comprise one or more of the following acts: mapping a bath site of the impurity-bath model onto a bath qubit using a fermion-to-qubit mapping transform; and mapping a fermionic Hamiltonian $H_{AIM}$ of the impurity-bath model onto a qubit Hamiltonian $\tilde{H}_{qubit}(V_p, \varepsilon_p, U)$ based on the fermion-to-qubit mapping transform. In some embodiments, the quantum circuit may be determined based on the qubit Hamiltonian $\tilde{H}_{qubit}(V_p, \varepsilon_p, U)$.

In one or more embodiments, the qubit Hamiltonian $\tilde{H}_{qubit}(V_p, \varepsilon_p, U)$ may be encoded into an encoded Hamiltonian, and the encoded Hamiltonian may be applied using a Trotterization procedure.

In one or more embodiments, the proposed method may further comprise: adding one or more additional fermionic sites to the (dissipative) impurity-bath model.

In one or more embodiments, for a bath site of index $p$ of one or more of the bath sites of the impurity-bath model, a corresponding jump operator $L_p$ may be modified into $L_p \gamma_i$, wherein the operator $\gamma_i$ acts on an additional fermionic site of index $i$ among the one or more additional fermionic sites is configured to perform the following operation:

$$\gamma_i = d_i + d_i^\dagger.$$

According to another aspect of the present subject disclosure, a quantum computer apparatus (e.g. configured for digital quantum computing) is proposed, which comprises a quantum processor, and a memory operatively coupled to the processor. The proposed apparatus is configured to perform, using the quantum processor, embodiments of a proposed method according to the present subject disclosure.

According to yet another aspect of the present subject disclosure, a (non-transitory) computer-readable medium encoded with executable instructions which, when executed on a non-quantum computer, causes a processor of the non-quantum computer, that is operatively coupled with a memory, to perform embodiments of a proposed method according to the present subject disclosure, is proposed.

According to yet another aspect of the present subject disclosure, a computer program product comprising computer program code (tangibly) embodied in a computer readable medium, said computer program code comprising instructions to, when provided to a non-quantum computer system and executed, cause said computer to perform embodiments of a proposed method according to the present subject disclosure, is proposed.

According to yet another aspect of the present subject disclosure, a computer program product comprising computer program code (tangibly) embodied in a computer readable medium, said computer program code comprising instructions to, when provided to a quantum computer system and executed, cause said computer to execute a quantum circuit for computing a Green's function of a solid-state material sample corresponding to an impurity-bath model as part of performing embodiments of a proposed method according to the present subject disclosure, is proposed.

It should be appreciated that the present invention can be implemented and utilized in numerous ways, including without limitation as a process, an apparatus, a system, a device, and as a method for applications now known and later developed. These and other unique features of the system disclosed herein will become more readily apparent from the following description and the accompanying drawings.

## Brief description of the drawings

The present subject disclosure will be better understood and its numerous objects and advantages will become more apparent to those skilled in the art by reference to the following drawings, in conjunction with the accompanying specification, in which:

Figure 1 illustrates some aspects of proposed methods in accordance with one or more embodiments;
Figure 2 illustrates the performance of a fitting procedure, in accordance with one or more embodiments;
Figure 3 illustrates the use of noise to capture the Kondo effect, in accordance with one or more embodiments;
Figure 4 illustrates an exemplary heterogeneous quantum architecture, in accordance with one or more embodiments;
Figure 5 illustrates quantum operators that may be used in accordance with one or more embodiments;
Figure 6 is a block diagram that illustrates a proposed method, in accordance with one or more embodiments;
Figure 7 is a block diagram that illustrates a proposed method, in accordance with one or more embodiments;
Figure 8a is a diagram that illustrates an exemplary iterative process for computing a Green's function of a solid state material, in accordance with one or more embodiments;
Figure 8b is a diagram that illustrates an exemplary scheme for a Green's function of an impurity, in accordance with one or more embodiments;
Figure 8c illustrates an exemplary quantum circuit for encoding qubit noise into fermion noise, in accordance with one or more embodiments;
Figure 8d illustrates a quantum circuit simplification that may be used in accordance with one or more embodiments;
Figure 8e illustrates an exemplary quantum circuit, in accordance with one or more embodiments;
Figure 8f illustrates an exemplary quantum circuit that may be used in accordance with one or more embodiments;
Figure 9 illustrates an exemplary apparatus configured to implement a proposed method, in accordance with one or more embodiments.

## Description of embodiments

The advantages, and other features of the components disclosed herein, will become more readily apparent to those having ordinary skill in the art form. The following detailed description of certain preferred embodiments, taken in conjunction with the drawings, sets forth representative embodiments of the subject technology, wherein like reference numerals identify similar structural elements.

For simplicity and clarity of illustration, the drawing figures illustrate the general manner of construction, and descriptions and details of well-known features and techniques may be omitted to avoid unnecessarily obscuring the discussion of the described embodiments of the subject disclosure. Additionally, elements in the drawing figures are not necessarily drawn to scale. For example, the dimensions of some of the elements in the figures may be exaggerated relative to other elements to help improve understanding of embodiments of the present subject disclosure. Certain figures may be shown in an idealized fashion in order to aid understanding, such as when structures are shown having straight lines, sharp angles, and/or parallel planes or the like that under real-world conditions would likely be significantly less symmetric and orderly.

In addition, it should be apparent that the teaching herein can be embodied in a wide variety of forms and that any specific structure and/or function disclosed herein is merely representative. In particular, one skilled in the art will appreciate that an aspect disclosed herein can be implemented independently of any other aspects and that several aspects can be combined in various ways.

The present disclosure is described below with reference to functions, engines, block diagrams and flowchart illustrations of the methods, systems, and computer program according to one or more exemplary embodiments. Each described function, engine, block of the block diagrams and flowchart illustrations can be implemented in hardware, software, firmware, middleware, microcode, or any suitable combination thereof. If implemented in software, the functions, engines, blocks of the block diagrams and/or flowchart illustrations can be implemented by computer program instructions or software code, which may be stored or transmitted over a computer-readable medium, or loaded onto a general purpose non quantum computer, special purpose non quantum computer or other programmable data processing apparatus to produce a machine, such that the computer program instructions or software code which execute on the non quantum computer or other programmable data processing apparatus, create the means for implementing the functions described herein.

Embodiments of computer-readable media includes, but are not limited to, both computer storage media and communication media including any medium that facilitates transfer of a computer program from one place to another. As used herein, a "computer storage media" may be any physical media that can be accessed by a computer. Examples of computer storage media include, but are not limited to, a flash drive or other flash memory devices (e.g. memory keys, memory sticks, key drive), CD-ROM or other optical storage, DVD, magnetic disk storage or other magnetic storage devices, memory chip, RAM, ROM, EEPROM, smart cards, Solid State Drive (SSD) devices or Hard Disk Drive (HDD) devices, or any other suitable medium from that can be used to carry or store program code in the form of instructions or data structures which can be read by a computer processor. Also, various forms of computer-readable media may transmit or carry instructions to a computer, including a router, gateway, server, or other transmission device, wired (coaxial cable, fiber, twisted pair, DSL cable) or wireless (infrared, radio, cellular, microwave). The instructions may comprise code from any computer-programming language, including, but not limited to, assembly, C, C++, Visual Basic, HTML, PHP, Java, Javascript, Python, Julia, and bash scripting.

Unless specifically stated otherwise, it will be appreciated that throughout the following description discussions utilizing terms such as processing, computing, calculating, determining, generating, or the like, refer to the action or processes of a computer or computing system, or similar electronic computing device, that manipulate or transform data represented as physical, such as electronic, quantities within the registers or memories of the computing system into other data similarly represented as physical quantities within the memories, registers or other such information storage, transmission or display devices of the computing system.

The terms "comprise," "include," "have," and any variations thereof, are intended to cover a non-exclusive inclusion, such that a process, method, article, or apparatus that comprises a list of elements is not necessarily limited to those elements, but may include other elements not expressly listed or inherent to such process, method, article, or apparatus.

The terms "quantum" or "quantum computing" as used in the present subject disclosure are intended to cover any computer, computing system, processing or computing operation, configured to use or exploit quantum mechanical phenomena. A computer, processor, calculator, computing system, computing node, computing task, computer job, processing, algorithm, and processing resource configured to use or exploit quantum mechanical phenomena will be referred to herein as "quantum" (a quantum computer, quantum processor, quantum calculator, quantum computing system, quantum computing node, quantum computing task, quantum computer job, quantum processing, quantum algorithm, and quantum processing resource, respectively). In contrast, a computer, processor, calculator, computing system, computing node, computing task, computer job, processing, algorithm, and processing resource which is not configured to use or exploit quantum mechanical phenomena may be referred to herein as "classical" or "non-quantum" (a classical computer, classical processor, classical calculator, classical computing system, classical computing system, classical computing node, classical computing task, classical computer job, classical algorithm, classical processing, classical processing resource, respectively). A quantum processor (also referred to herein as a quantum processing unit (or QPU)) may be configured to perform both quantum processing and classical processing.

The terms "digital quantum computing" as used in the present subject disclosure refers to quantum computing that involves a logic gate-based description of a computer program, as opposed to a continuous-time (also referred to as "analogue") description. The terms "digital quantum processor", and "digital quantum computer" respectively refer to a quantum processor and a quantum computer configured to perform digital quantum computing.

The term "hybrid" as used in the present subject disclosure, for example as applied to a computer, an algorithm, a computer task, a computer job, refers to the combination of classical and quantum.

Additionally, the word "exemplary" as used herein means serving as an example, instance, or illustration. Any aspect or design described herein as "exemplary" is not necessarily to be construed as preferred or advantageous over other aspects or designs.

The present subject disclosure may advantageously be implemented on any suitable computing environment comprising a quantum computer equipped with quantum processing resources (e.g. one or more QPUs, such as for example a superconducting processor, a trapped-ion processor, or a neutral atoms processor). For example, the present subject disclosure may be implemented on a hybrid computer configured with one or more classical resources (e.g. one or more CPUs) and one or more quantum processing resources (e.g. one or more QPUs), such as a hybrid HPC cluster, an electronic component, an electronic chipset, a QPU, an electronic circuitboard, an electronic circuit, etc.

In the following, embodiments of the present subject disclosure are described in the exemplary case of a determination of a quantum phase parameter characterizing a property of a quantum phase of a crystal sample which relates to the quasiparticle weight of the crystal sample. However, a person of ordinary skill in the art would understand that the processes, apparatuses and computer programs of the present subject disclosure may be implemented for determining a quantum phase parameter characterizing any suitable property of any solid-state material sample, and that such proposed processes, apparatuses and computer programs of the present subject disclosure are not limited to the determination of a quantum phase parameter characterizing a property of a quantum phase of a crystal sample or to the determination of a quantum phase parameter characterizing a property of a quantum phase of a crystal sample related to the quasiparticle weight of the crystal sample, which is provided as an example only.

Dynamical Mean Field Theory (DMFT) is widely used for investigating, studying or designing strongly-correlated electron systems, thanks to its ability to capture Fermi-liquid as well as Mott physics, a feature not shared by other simpler embeddings like Density-Matrix Embedding Theory (DMET). This comes at a price: the dynamical character of DMFT is embodied by the frequency dependence of DMFT's so-called "hybridization function" (denoted $\Delta(\omega)$ ). While quantum Monte-Carlo approaches can directly deal with the full frequency dependence thanks to the path-integral formalism, they suffer from the so-called "infamous sign problem" both at and out of equilibrium. This has prompted efforts to use quantum processors as coprocessors to solve the impurity-bath model.

To this aim, the impurity-bath model may advantageously be formulated with auxiliary degrees of freedom - usually referred to as "bath sites", with energies $\varepsilon_p$ and couplings $V_p$ - are introduced to mimic the frequency dependence of the hybridization, leading to a hybridization function of the frequency $\omega$ of the following form:

$$\Delta(\omega) = \sum_{p=0}^{\infty} \frac{V_p^2}{\omega - \varepsilon_p}$$

(Eq. 1)

In practical numerical computations, the (theoretically infinite) number of bath sites (of index $p$ in Eq. 1) is usually truncated to a finite value ($N_b$), which introduces a truncation error.

On classical computers, the number of bath sites can be pushed to quite large values, however arguably not enough to capture long-time dynamics in an out-of-equilibrium setting. As a consequence, it may advantageously be contemplated to transfer the burden of reaching larger numbers of baths to quantum processors, which do not suffer from an exponential scaling of the computational cost with $N_b$.

One main issue of previous attempts to solve an impurity problem with a finite yet large number of baths on a quantum computer is that the number of bath sites is, via fermion-to-spin transforms (such as for example the Jordan-Wigner transformation), directly linked (and in some cases equal) to the number of qubits required on the quantum computer.

Beyond the above-mentioned issue regarding the number of qubits required to perform the computations for solving an impurity problem on a quantum computer, today's Noisy Intermediate-Scale Quantum (NISQ) processors are limited by decoherence, which places a drastic requirement on the number of quantum operations that can be executed with an acceptable level of errors.

In addition, a large number of bath sites also means a larger number of gates needed to entangle these qubits, that is, computations implemented on a quantum computer with an increased computation complexity.

Therefore, an implementation of DMFT with an acceptable truncation error, and thus a large enough number of bath sites appears to be a technical challenge. Such challenge is advantageously addressed by the proposed schemes of the present subject disclosure.

As is described below in further details, proposed schemes of the present subject disclosure advantageously leverage in some embodiments the quantum noise which is inherent to NISQ quantum computers in order to reduce the number of qubits required to achieve a hybrid quantum-classical DMFT computation.

In particular, some proposed schemes of the present subject disclosure use the broadening of bath levels brought forth by a particular class of noise in order to reduce the number of requisite bath sites for fitting the hybridization function.

## *Model and method:*

One may consider the Hubbard model on a Bethe lattice, which may constitute a simplified model for a solid-state material sample. It is defined by the following Hamiltonian:

$$H_{Hubbard} = -t \sum_{i,j,\sigma} (c_{i\sigma}^+ c_{j\sigma} + c_{j\sigma}^+ c_{i\sigma}) + U \sum_i n_{i\uparrow} n_{i\downarrow}$$

(Eq. 2)

where $t$ designates the nearest-neighbor hopping strength and $U$ the Hubbard interaction strength. The indices $i,j$ denote lattice sites of the Hubbard model, $\sigma \in \{\uparrow, \downarrow\}$ denotes electron spin, and $c_{i\sigma}^+$ and $c_{i\sigma}$ respectively denote spin $\sigma$ electron creation and annihilation operators on the ith lattice site, with $n_{i\sigma} = c_{i\sigma}^+ c_{i\sigma}$.

Even though this model is often considered as one of the simplest models for describing strongly correlated materials, it is nevertheless hard to solve on classical computers. The dynamical mean field theory (DMFT) method was introduced in order to reduce the computational complexity of the Hubbard model by (self-consistently) mapping it to a so-called "impurity-bath model," as described below in further details.

## *DMFT without dissipation*

For example, in some embodiments, the above Hubbard model on a Bethe lattice may be solved with a conventional DMFT method that assumes no dissipation.

In a simple version of a Bethe lattice ( which leads to an impurity model with only one interacting site), solving this model (i.e. determining an impurity Green's function of frequency $\omega$ (denoted $G_{imp}(\omega)$) that corresponds to the adopted Hubbard model) may involve an iterative process that leads through convergence to the sought impurity Green's function.

Such an iterative process may typically be initialized using an initial impurity Green's function $G_{imp,0}(\omega)$ (which may be predefined).

In this iterative process, one or more iterations of index $j$ may comprise one or more of the following acts:

- computing a hybridization function $\Delta_j(\omega)$ based on the impurity Green's function $G_{imp,k-1}(\omega)$ obtained from the previous iteration of index $j$ - 1 (e.g. via $\Delta_j(\omega) = t^2 G_{imp,k-1}(\omega)$),
- based on the computed hybridization function $\Delta_j(\omega)$, determining (e.g. computing) an Anderson impurity-bath model's action (denoted $S_{imp}$), for example using the following equation:

$$S_{imp}(c_\sigma^*, c_\sigma) = \iint d\tau d\tau' \sum_\sigma c_\sigma^*(\tau)\Delta(\tau - \tau')c_\sigma(\tau') + \int d\tau U n_\uparrow(\tau) n_\downarrow(\tau)$$

(Eq. 3).

Since this impurity-bath model only includes one interacting site, it is simpler to solve than the original Hubbard model (where all the sites are deemed interacting).

In the following description related to this simple impurity-bath model, the site index i is therefore dropped.

- The Green's function $G_{imp,k}(\omega)$ can then be computed for the current iteration $j$, for example based on a Fourier transform of $G_{imp}(\tau) = - \int \mathcal{D}[c, c^*]c(\tau)c^*(0)_e{}^{-S_{imp}}$.

The Green's function $G_{imp,k}(\omega)$ computed at the current iteration $j$ can then be used as a new starting point to the next iteration of the iterative process.

*Hamiltonian formulation of the Anderson impurity-bath model's action ($S_{imp}$):*

In order to use a quantum processor for performing computations of an iteration using a quantum processor (instead of a classical processor), a Hamiltonian formulation of the used Anderson impurity-bath model's action ( $S_{imp}$ ) may advantageously be achieved.

For example, one may use the Anderson impurity Hamiltonian, expressed as follows as an auxiliary model:

$$H_{AIM} = U n_\uparrow n_\downarrow + \sum_\sigma \sum_{p=0}^{\infty} \{\varepsilon_p a_{p\sigma}^\dagger a_{p\sigma} + V_p(c_\sigma^\dagger a_{p\sigma} + a_{p\sigma}^\dagger c_\sigma)\}$$

(Eq. 4)

where $a_{p\sigma}^\dagger$ and $a_{p\sigma}$ respectively denote spin $\sigma$ electron creation and annihilation operators on the bath site *p* of the impurity-bath model, and $c_\sigma^\dagger$ and $c_\sigma$ respectively denote spin $\sigma$ electron creation and annihilation operators on the impurity of the impurity-bath model.

The above Anderson impurity Hamiltonian ($H_{AIM}$) describes an impurity coupled to bath sites of energy $\varepsilon_p$ with coupling strength $V_p$, and does not explicitly use the hybridization function $\Delta(\omega)$.

Upon integrating the bath degrees of freedom, respectively represented by creation and annihilation operators $a_{p\sigma}^\dagger$ and $a_{p\sigma}$, the Anderson impurity Hamiltonian is equivalent to the original action formulation, so that the impurity-bath model can be represented in an equivalent manner by the Anderson impurity Hamiltonian ($H_{AIM}$) or by the Anderson impurity-bath model's action ($S_{imp}$):

As the above Anderson impurity Hamiltonian ($H_{AIM}$) describes an impurity coupled to bath sites of energy $\varepsilon_p$ with coupling strength $V_p$, without explicitly using the hybridization function $\Delta(\omega)$, in contrast with the above action (e.g. creation, annihilation) which explicitly uses the hybridization function $\Delta(\omega)$, a condition for representing the impurity-bath model in an equivalent manner by the Anderson impurity Hamiltonian ($H_{AIM}$) or by the Anderson impurity-bath model's action ($S_{imp}$) may be expressed based on Eq. (1) being satisfied (so that the energy $\varepsilon_p$ with coupling strength $V_p$ parameters used in the Hamiltonian formulation correspond the hybridization function used in the action formulation).

In practice, Eq. 1 is typically only solved in an approximate fashion because the number of sites ($N_b$) cannot be infinite:

$$\Delta(\omega) \approx \sum_{p=0}^{N_b-1} \frac{V_p^2}{\omega - \varepsilon_p}$$

(Eq. 5)

In one or more embodiments, the Hamiltonian formulation ($H_{AIM}$) may then be considered equivalent to the action formulation ($S_{imp}$) as long as Eq. 5 is verified.

*Fitting procedure:*

With respect to verifying Eq. 5, as shown by Eq. 5, determined estimates of the parameters $V_p$ and $\varepsilon_p$ (for each bath of index *p*) may be used in some embodiments to determine an estimate of (approximate) the hybridization function $\Delta(\omega)$.

In the present subject disclosure, the determination of an estimate (an approximation) of the hybridization function $\Delta(\omega)$ using Eq. 5, that is, based on estimates of the parameters $V_p$ and $\varepsilon_p$ (for each bath of index *p*), is referred to herein as the "fitting operation" or the "fitting procedure".

The truncation error may be measured, for example, by the distance $\varepsilon$ expressed as:

$$\epsilon = \left\| \int d\omega W(\omega) \left[ \Delta(\omega) - \sum_{p=0}^{N_b-1} \frac{V_p^2}{\omega - \varepsilon_p} \right] \right\|$$

with $W(\omega)$ an arbitrary positive weighting function, which goes to zero as $|\omega|$ goes to large values.

Large values of $N_b$ (typically 50) lead to intractable impurity-bath models on classical processors owing to the exponential size of the corresponding Hilbert space.

On the other hand, current quantum processors come with a small number of usable qubits, in the following sense: The number of qubits that can be entangled before decoherence sets in is limited, so that conventional attempts to solve an impurity-bath model with a hybrid quantum-classical scheme have been limited to one impurity and one bath site and more recently, thanks to a tailored orbital optimization procedure, to only two impurities and two bath sites.

_Adding dissipation to the bath: DMFT with dissipation:_

Given that computations performed on a quantum computer for each bath site of the used model would require at least one qubit, a dissipative Anderson impurity-bath model is advantageously used in one or more embodiments in order to relieve the requirement on the number of bath sites, hence decrease the number of required qubits for performing computations for all of the bath sites of the model.

A dissipative impurity Anderson model which includes bath sites that are subject to dissipation may therefore be used in one or more embodiments.

For example, assuming a Markovian environment, a dissipative Anderson impurity-bath model that is equivalent to the above-described (Eq. 4) Hamiltonian model may be described by the following Lindblad equation for the density matrix:

$$i\frac{d\rho}{dt} = [H_{AIM},\rho] + i\sum_{p,\sigma}\left(L_{p\sigma}\rho L_{p\sigma}^\dagger - \frac{1}{2}\{L_{p\sigma}^\dagger L_{p\sigma},\rho\}\right)$$

[Eq. 6]

where $p$ is the bath site index, $\sigma$ represents (electron) spin, and $\rho$ represents the density matrix of the system. The symbols "{.,.}" (respectively "[.,.]") denotes an anticommutator matrix operator ($\{a, b\} = a.b + b.a$) (respectively a commutator matrix operator ($[a,b] = a.b - b.a$)). In Eq. 6, the matrix $L_{p\sigma}$ designates a jump operator acting on bath site $p$ and spin $\sigma$ to represent production of dissipation occurring on the bath site $p$, and $L_{p\sigma}^\dagger$ designate the adjoint (Hermitian conjugate) of the matrix $L_{p\sigma}$.

The noise (for example generated, from the standpoint of the system, from the environment external to the system) is described by the jump operators $L_{p\sigma}$ and $L_{p\sigma}^\dagger$.

In principle, the jump operators $L_{p\sigma}$ and $L_{p\sigma}^\dagger$ act on all degrees of freedom, i.e. correlated impurities and bath sites alike.

In one or more embodiments, in order to use the model of Eq. 6 for solving the impurity-bath model, the jump operators $L_{p\sigma}$ and $L_{p\sigma}^\dagger$ may advantageously be chosen so that Eq. 6 corresponds to the impurity-bath model to be solved.

In some embodiments, one may define (determine) jump operators $L_{p\sigma}$ and $L_{p\sigma}^\dagger$ that act only on the bath sites. For example, in some embodiments, jump operators $L_{p\sigma}$ and $L_{p\sigma}^\dagger$ having the following form may be used:

In some embodiments, bath sites may be split in two groups: for example, bath sites of even index may be referred to as "emitters", and bath sites of odd index may be referred to as "absorbers".

In some embodiments, a corresponding set of jump operators may be defined with the same noise level $\Lambda$ (which may be defined as the rate at which the jump operators are applied), for example as follows:

$$L_{2q,\sigma} = \sqrt{\Lambda}a_{2q,\sigma}^\dagger \text{ (emitters bath sites) (Eq. 7\_1),}$$

$$L_{2q+1,\sigma} = \sqrt{\Lambda}a_{2q+1,\sigma} \text{ (absorbers bath sites) (Eq. 7\_2),}$$

_for a = 0,..., ($N_b$ - 1)/2._

The present subject disclosure provides schemes that advantageously recognize that a dissipative model (for example represented by the above-stated Lindblad equation (Eq. 6)) of an impurity problem may also represent the operating of a noisy quantum computer.

As discussed above, the dissipative model of the impurity problem may in some embodiments be represented using the above Lindblad equation for which jump operators have been defined to correspond to the impurity problem.

Such a Lindblad equation with specific jump operators can advantageously also represent the operating of a noisy quantum computer, so that solving the impurity problem can use, according to schemes of the present subject disclosure, both non-noisy qubits and noisy qubits of the noisy quantum computer.

In one or more embodiments, this dissipative process can advantageously be viewed as corresponding to the functioning of a model quantum computer (e.g. a model fermionic quantum computer) where one or a few (fermionic) modes are deemed "perfect" (dissipation free) and most other (fermionic) modes are "noisy" (for example either because they emit extra fermions or absorb them).

This advantageously leads, according to schemes of the present subject disclosure, to match parameters of this dissipative process model to operations of a quantum computer through implementation of the model quantum computer after encoding of the dissipative process on a quantum computer (e.g. with quantum gates).

In one or more embodiments, solving the above Lindblad equation (Eq. 6) (with jump operators chosen so that the equation corresponds to the impurity problem) requires determining the (Anderson impurity) Hamiltonian ($H_{AIM}$), which as described above may comprise the determination of respective estimates of the parameters $V_p$ and $\varepsilon_p$ (for each bath of index $p$) for each bath site of the impurity-bath model that match the hybridization function (so that Eq. (5) for a finite number of bath sites be verified).

In one or more embodiments, a fitting procedure such as described above for these parameters $V_p$ and $\varepsilon_p$ may be used for that purpose.

In one or more embodiments, a fitting of the hybridization function that takes into account the noise parameter $\Lambda$ (in addition to the $V_p$ and $\varepsilon_p$ parameters) may be performed based on a splitting between emitters and absorbers, such as for example the splitting described above (Eqs. 7_1 and 7_2).

It can be shown that creation (resp. annihilation) jump operators contribute only to the lesser (resp. greater) steady state Green's function. As a result, in some embodiments, one can advantageously fit these two components separately: the $\varepsilon_{2k}$ and $V_{2k}$ are chosen to fit the lesser Green's function, while the $\varepsilon_{2k+1}$ and $V_{2k+1}$ address the greater Green's function.

In some embodiments, it may be considered that $\Lambda$ is a given noise level parameter that represents the quality of the (e.g. fermionic) quantum processor used to perform the computations for solving the impurity-bath model.

In embodiments in which this noise level $\Lambda$ is constant, the fitting procedure may determine estimates of the parameters $V_p$ and $\varepsilon_p$ (for each bath of index $p$), with no need to determine an estimate of $\Lambda$.

In embodiments in which this noise level $\Lambda$ is variable, the fitting procedure may determine estimates of the parameters $\Lambda$, and $V_p$ and $\varepsilon_p$ (for each bath of index $p$).

Regardless of whether the noise level is considered constant or variable, given the above-described jump operators (absorbers and emitters of Eq. 7a and 7b), the integration of the bath degrees of freedom modifies the retarded hybridization function of Eq. (5) to the following retarded hybridization function:

$$\Delta(\omega) = \sum_{p=0}^{N_b-1} \frac{V_p^2}{\omega - \varepsilon_p + i\Lambda}$$

(Eq. 8)

that corresponds to the following lesser and greater hybridization functions:

$$\Delta^{<}(\omega) = 2i \sum_{q=0}^{(N_b-1)/2} \frac{V_{2q}^2 \Lambda}{\left|\omega - \varepsilon_{2q} + i\Lambda\right|^2}$$

(Eq. 9-1)

$$\Delta^>(\omega) = -2i \sum_{q=0}^{(N_b-1)/2} \frac{V_{2q+1}^2 \Lambda}{\left|\omega - \varepsilon_{2q+1} + i\Lambda\right|^2}$$

(Eq. 9-2)

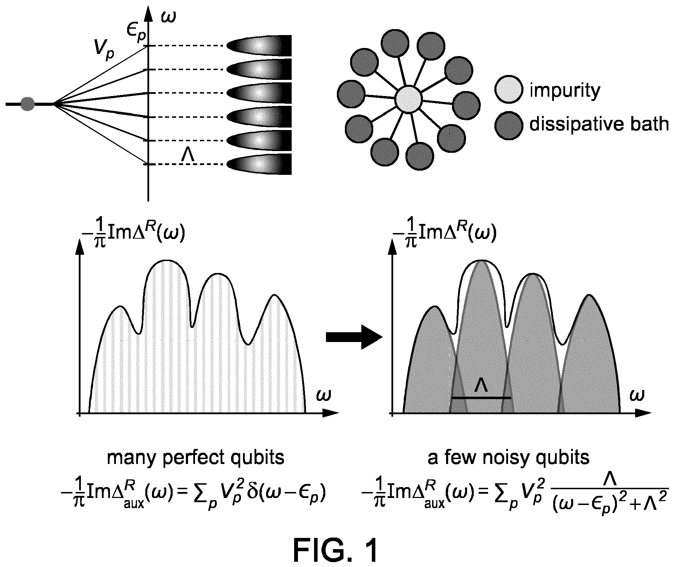

Each bath site now corresponds to a Lorentzian peak of width $\Lambda$, either in the lesser or the greater component, instead of an infinitesimally thin Dirac peak, as illustrated in Fig. 1.

In one or more embodiments, respective fitting procedures may be applied for the lesser hybridization function and the greater hybridization function implemented to determine the $V_p$ and $\varepsilon_p$ so that Eqs 9-1 and 9-2 hold.

Fig. 1 illustrates principles of schemes proposed in the present subject disclosure.

As shown in Fig. 1, using a few bath sites with dissipation strength $\Lambda$ (upper left), corresponding to dissipation-less impurity sites and dissipative bath sites (upper right), one can fit a hybridization function with fewer bath levels than in the absence of dissipation (lower panels).

The present subject disclosure advantageously provides schemes that use this noise-induced broadening to decrease the number of bath levels needed to reach a given accuracy $\varepsilon$ compared to the usual, noiseless case.

*Results:*

In this section, we explore what advantage can be reached with proposed schemes of the present subject disclosure for the exemplary case of an Anderson impurity-bath model defined as in Eq. (4).

We first study quantitatively how close noisy bath qubits can reproduce the target hybridization function. We then show a clear advantage in using such a dissipative bath on the case of obtaining the Kondo temperature, a key quantity for the physics of these models.

*Advantage for the quality of the fit:*

Using noisy qubits as proposed in some embodiments brings an advantage for representing accurately the hybridization function, as described below in further details:

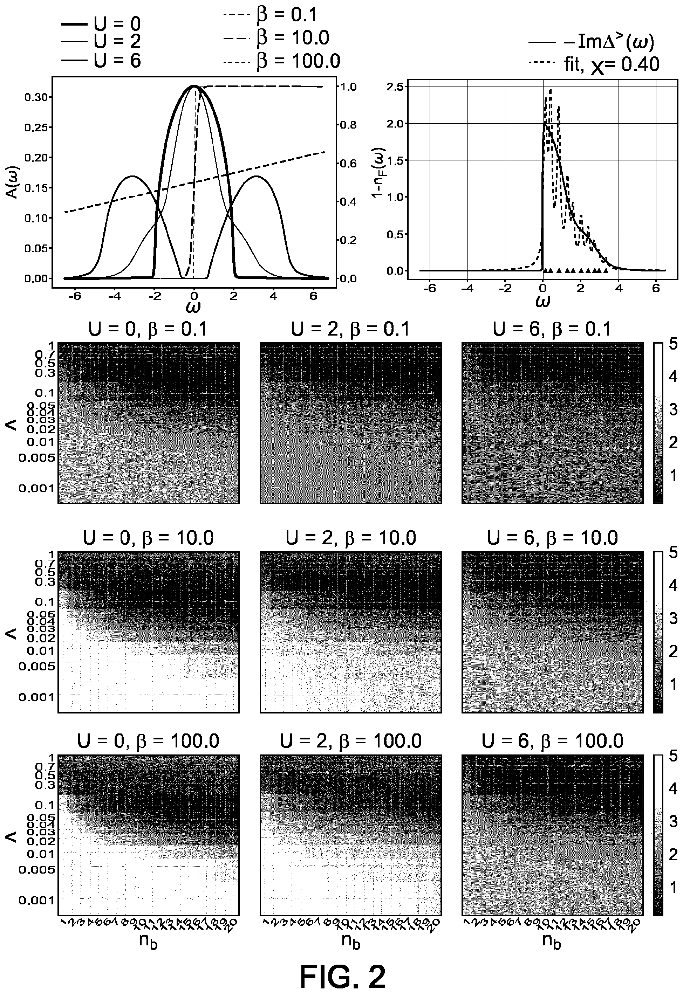

Fig. 2 illustrates the performance of the fitting procedure for various hybridization function shapes corresponding to decreasing temperatures and shown in the top left panel (which shows the exemplary hybridization functions which were considered to test the quality of the fit). An example of such a fit can be seen in the top right panel. In the nine lower panels, shown is the fitting error (warm is more error, dark is more accurate) (fit quality $\varepsilon$ for various $(U, \beta)$) as a function of the number of bath levels $N_b$ (number of bath sites) and of the noise strength $\eta$, for different hybridization functions (increasing $U$ going to the right, decreasing temperature going downward).

As illustrated by Fig. 2, the noiseless case is reached in the limit $\Lambda \to 0$. It is clear from Fig. 2 that, at a fixed number of qubits, increasing noise from this limit systematically reduces the error, and thus brings a clear advantage.

Furthermore, one can see that for high temperatures, where the features of the hybridization function are quite broad, increasing noise does yield a more accurate fit using fewer bath sites. At lower temperatures, as expected, the sharp features associated with the Fermi-Dirac distribution cannot be resolved anymore starting from a given noise strength: for the fitting procedure to work, the noise strength must be low enough so that it can resolve fine structures.

At large $U$, the hybridization function has a shape relevant for an insulating material, with a gap at the Fermi level. In that case, a low temperature is not an issue anymore, and even a strong noise produces a good fit with a small number of qubits (bottom right panel of Fig. 2).

*Advantage for capturing many-body effects:*

In the following, we show that, in addition to bringing an advantage in terms of the quality of the fit of the hybridization function, proposed schemes according to the present subject disclosure also bring an advantage for the computation of quantities of interest, such as the so-called "Kondo resonance" property.

We take the example of the Kondo resonance, a feature of the Anderson impurity-bath model which is a purely many-body phenomenon, and thus particularly difficult to compute. The Kondo resonance is a characteristic feature of the spectral function $A(\omega)$, defined as $A(\omega) = -1/\pi Im G_{imp}^R(\omega)$ in the so-called "Kondo regime".

In one or more embodiments, using only a few (4) noisy qubits to represent the bath may advantageously allow to recover (e.g. to compute, or to estimate) the Kondo resonance, whereas a large number of qubits would be necessary in the absence of noise, due to the many-body nature of the Kondo resonance feature.

In one or more embodiments, in order to compute the spectral function $A(\omega)$ and thus observe the Kondo resonance, the impurity-bath model's real time Green's function $G_{imp}(t)$ may be computed on a noisy quantum computer.

This computation may be emulated by a classical time evolution of the Lindblad equation Eq. (6). Such an evolution can be shown to yield the Green's function by virtue of the so-called quantum regression theorem, as described in more detail in Appendix A.

To obtain fine spectral structures in the spectral function, long time dynamics of the Green's function are necessary in the absence of dissipation. This in turn requires large baths to avoid unwanted revivals due to the finite size of the bath.

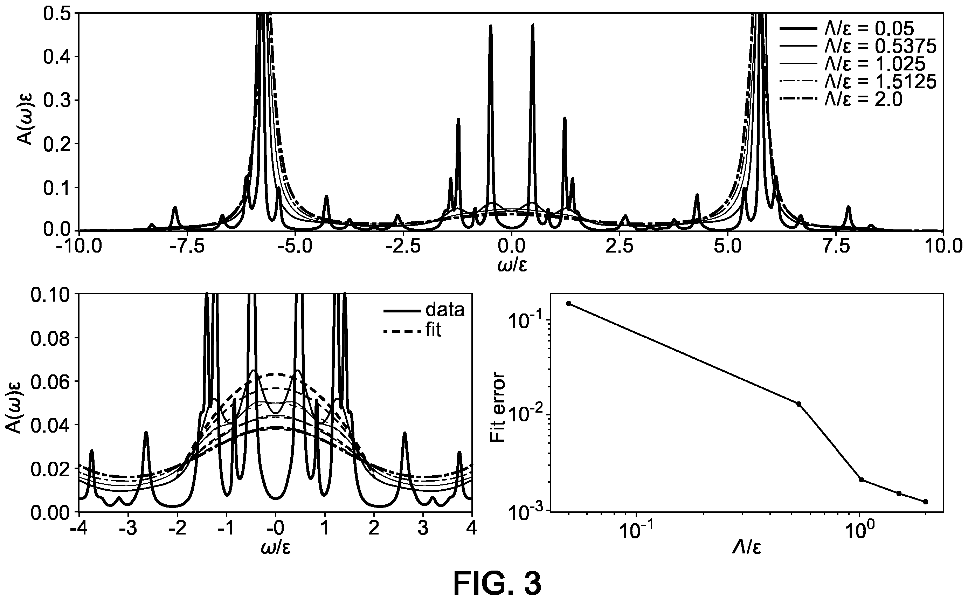

In contrast, in the presence of dissipation, a small bath size may advantageously be used to identify the Kondo resonance. This is shown in Figure 3, which compares the spectra obtained at different strengths of dissipation in an Anderson impurity-bath model with only $N_b = 2$ bath sites. The higher energy bath site is an absorber and is placed at energy $\varepsilon$, while the lower bath site is an emitter and is placed at energy $-\varepsilon$. This is meant to approximate a thermal bath with a temperature of the order of $\varepsilon$: the emitter site emulates the low-energy occupied part of a Fermi sea, and the absorber emulates the high-energy empty part.

Fig. 3 illustrates the use of noise to capture the Kondo effect.

The top panel of Fig. 3 shows an exemplary spectral function for the Anderson impurity-bath model in the exemplary case of $N_b = 2$ bath sites for varying noise level $\eta$. The bottom left panel of Fig. 3 shows a zoom on the central resonance and fit to Lorentzian functions. The bottom right panel of Fig. 3 shows the error in a Lorentzian fit to the spectral function central peak, for increasing noise level $\Lambda$. As shown by Fig. 3, the Kondo resonance is increasingly well captured as the noise level increases.

Couplings between bath sites and the impurity are $V_p = \varepsilon$ and the impurity Coulomb interaction is $U = 10\varepsilon$, as a strong repulsion is necessary to witness the Kondo effect.

To keep particle-hole symmetry, the impurity energy level is set at $-U/2$.

At large noise strength $\Lambda$, the spectrum shows a characteristic three peak structure: two symmetric peaks at energies $\omega \approx \mp U/2$ corresponding to transitions between the singly occupied state and the empty or fully occupied states; and a central peak at $\omega = 0$ corresponding to the Kondo resonance. As $\Lambda$ decreases, the spectrum gets closer to the discrete spectrum of the dissipation-less system, and the Kondo resonance gets broken down to a Dirac comb.

Therefore, attempting to obtain the width of the Kondo resonance (the so-called "Kondo temperature") becomes increasingly difficult as $\Lambda$ decreases. Fits to a Lorentzian function can be seen in the bottom left panel of Fig. 3, which zooms in the lower energies. The error made by these fits is shown in the bottom right panel, and we observe a clear improvement at stronger dissipation strengths.

The exemplary simplified bath model used for generating the curves of Fig. 3 (two bath sites) allows observing some characteristic behavior of the Kondo effect: As $\Lambda$ increases, the effective temperature of the simplified bath model increases ($\Delta<(\omega)$ and $\Delta>(\omega)$ overlap more and more), and this is expected to reduce the height of the Kondo resonance. This is indeed what we observe (bottom left panel of Fig. 3).

In addition to requiring fewer qubits, using noisy qubits according to embodiments of the present subject disclosure yields another advantage: the initial state of the system under study does not need to be set algorithmically, but is the natural steady state resulting from the evolution under dissipation of Eq. (6). In the noiseless case, computing Green's functions requires to prepare the quantum register in the ground state of the Hamiltonian (e.g. Eq. (4)). This comes at the cost of applying a preprocessing quantum circuit, with potential errors.

In the noisy case as proposed in embodiments of the present subject disclosure, a steady state needs to be reached before evaluating the Green's function, but emerges automatically under the Hamiltonian evolution with noisy qubits.

*Implementation on a quantum computer:*

In the following, an exemplary implementation of the above-described model on current qubit-based quantum processors according to embodiments of the present subject disclosure is described.

The two above-mentioned advantages can be used to implement a DMFT computation on a quantum computer according to embodiments of the present subject disclosure.

Indeed, a typical DMFT loop, in the intermediate interaction regime, yields impurity spectra $A(\omega)$ with a Kondo resonance that, in a DMFT context, bears witness to the competition between Fermi-liquid physics (that favors electronic itinerancy, quantified with the weight of the central peak) and Mott physics (that favors electronic localization, and appears in the side bands).

As a by-product, the hybridization functions that need to be fitted within the self-consistent loop also acquires several peaks, which need to be resolved during the fitting procedure. Thus, the proposed noise-assisted schemes of the present subject disclosure, which allow for an enhancement of the fit quality in certain regimes, is also advantageously beneficial to the DMFT loop.

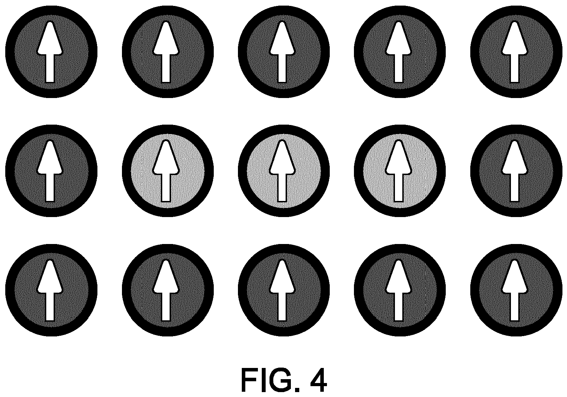

In one or more embodiments, in order to obtain the sought-after enhancements, a heterogeneous quantum processor architecture with a few noiseless qubits and a large number of noisy qubits such as illustrated by Fig. 4, may be used.

Fig. 4 shows an exemplary heterogeneous quantum architecture that may be used according to embodiments of the present subject disclosure. Fig. 4 illustrates an exemplary quantum processor with a few (in the example, 3) noiseless (or error-corrected) qubits, in light grey, and a few (in the example, 12) noisy qubits, in dark grey. In the present subject disclosure, the terms "perfect", "noiseless", "noise-free" or "error-corrected" may be used interchangeably to designate qubits that are error-corrected, as opposed to qubits that are not error-corrected (which may be referred to in the present subject disclosure as "noisy" qubits).

In one or more embodiments, one or more noiseless qubits may be obtained by quantum error correction, which requires a lot of resources (in terms of physical qubits, in particular), and can thus be put in practice only for a few (about 10) logical qubits for current and near-term architectures.

Advantageously, as a large number of noisy qubits are already available in current quantum processors, with tens or even hundreds of qubits readily available on transmon, trapped-ion or Rydberg atom processors, such current quantum processors may be used for implementing embodiments of the present subject disclosure. Advantageously, according to embodiments of the present subject disclosure, the noise of these qubits may be leveraged to the advantage of the DMFT computation.

In one or more embodiments, the noisy qubits that are used for implementing embodiments of the present subject disclosure may not be the same, and noisy qubits with different noise levels may be used for implementing embodiments. For example, the fitting procedure used in some embodiments may be configured to attribute large-$\Lambda$ (large noise level) qubits to broad features of the hybridization functions, and weak-$\Lambda$ (weak noise level) qubits to thin features. This way, a variety of noise levels in the noisy qubits used for implementing embodiments of the present subject disclosure may actually be beneficial to reducing the total number of qubits required.

Green's functions can be computed on such a quantum computer by conventional methods relying on simulating a Hamiltonian evolution through Trotterization.

In the following, exemplary embodiments of the present subject disclosure are described in further details.

In one or more embodiments, an "impurity-bath model" with noisy bath sites is used. In particular, in some embodiments, such an impurity-bath model with noisy bath sites is solved using computations on a noisy quantum computer.

In the following, exemplary schemes for solving a noisy impurity problem using a noisy quantum processor according to embodiments of the present subject disclosure will be described. In some embodiments, solving the noisy impurity problem using a noisy quantum processor may be done at the expense of adding ancilla qubits, as will be described below.

In one or more embodiments, a quantum algorithm may be designed that simulates the following Lindblad dynamics:

$$\frac{d\rho}{dt} = \mathcal{L}(\rho) = \mathcal{U}(\rho) + \sum_p^M \mathcal{D}_p(\rho)$$

$$\mathcal{U}(\rho) = -i[H, \rho]$$

$$\mathcal{D}_p(\rho) = -\Gamma_p \left( \frac{1}{2}\{L_p^\dagger L_p, \rho\} - L_p \rho L_p^\dagger \right)$$

with $L_p$ a creation or an annihilation operator: $a_p$, $a_p^\dagger$. These operators are not local due to the antisymmetric character of fermions, so that it may be desirable to design an algorithm using only local operations.

In one or more embodiment, the initial impurity problem may be "Trotterized" in N steps, that is, the time evolution is split into small time slices, for example in some embodiments as follows (denoting $dt = t/N$):

$$\rho(t) = e^{t\mathcal{L}}(\rho_0) = [e^{dt\mathcal{U}} \circ e^{dt\mathcal{D}_1} \circ ... \circ e^{dt\mathcal{D}_M}]^N(\rho_0) + \mathcal{O}(dt^2)$$

In one or more embodiments, the operation $e^{dt\mathcal{D}_i}$ (index is henceforth dropped for clarity) may be realized, that is, implemented with quantum gates.

Indeed, this is a quantum channel that can be written:

$$e^{dt\mathcal{D}_\square}(\rho) = \rho - \frac{\Gamma dt}{2}\{L^\dagger L, \rho\} + \Gamma dt L\rho L^\dagger + \mathcal{O}(dt^2)$$

$$e^{dt\mathcal{D}_\square}(\rho) = \mathrm{K}_0\rho K_0^\dagger + \mathrm{K}_1\rho K_1^\dagger + \mathcal{O}(dt^2)$$

with two Kraus operators:

$$\mathrm{K}_0 = e^{-\frac{\Gamma dt}{2}L^\dagger L}$$

$$\mathrm{K}_1 = \sqrt{\Gamma dt}L$$

*Non-local algorithm:*

In one or more embodiments, the following algorithm may be used for applying this quantum channel. However, it is worth noting that such algorithm is non-local, because of the fermionic nature of $L$:
Introduce an ancillary qubit, for example prepared in the state 10).

Then apply the unitary

$$U_{c0} = e^{\sqrt{\Gamma dt}(S^+L - L^\dagger S^-)}$$

with $S^\pm$ the raising and lowering operators of the ancillary qubit.

Then, tracing over the ancillary qubit leads to the channel we want, up to $\mathcal{O}(dt^2)$:
This can be seen by expanding the trace, and seeing that the $\langle 0|...|0\rangle$ term yields $\mathrm{K}_0$, and that the $\langle 1|...|1\rangle$ term yields $\mathrm{K}_1$.

The trace is not an algorithmic step, but simply a mathematical observation about the reduced density matrix.

In some embodiments, one or more of the following two rules may be applied:

- The ancilla is reset to 10) for the next Trotter step.
- For the next quantum channels to be well defined, the system qubits are decorrelated from the ancilla at the beginning of each channel.

This means that in some embodiments one or more of a measurement and a reset may be made on the ancilla at the end of each Trotter step.

Alternatively, in other embodiments, if the ancillary qubit has been prepared in the 11) state, the same procedure may be used simply replacing the unitary coupling by

$$U_{c1} = e^{\sqrt{\Gamma dt}(LS^- - S^+L^\dagger)}$$

This is the direct application of the jump algorithm to a quantum algorithm. The fact that $U_{c0}$ and $U_{c1}$ are non-local in any standard fermionic mappings may be seen as an issue, which can however be overcome in some embodiments by making them local, as described below:

*Local algorithm*

Numerous fermionic mappings have been designed to represent parity conserving fermionic operators (such as quadratic terms) with local qubit operators. However, in some of the proposed schemes according to the present subject disclosure, only the creation or annihilation operators $c^\dagger$ and $c$ may be applied, which are not parity conserving.

Standard mappings represent these with non-local qubit operations, namely terms which are local in their fermionic representation become non-local, namely with a nonconstant support, in their qubit representation. This is a direct consequence of the fact that, when adding (or removing) a fermion on the mode of index $i$, the wavefunction gains a

sign $(-1)^n$ with $n = \sum_{j<i} n_j$ the number of fermions (of the same species) that are present in mode indexed by all $j < i$, in order to conserve antisymmetry.

In one or more embodiments, in order to make such qubit operations local, an additional ancillary fermionic mode $d$ may be used.

When we want to add a fermion on the annihilation operator $c$, we instead do:

- if $d$ is empty, add a pair of fermions: one on $c$ and one on $d$
- if $d$ is full, move the fermion from $d$ to $c$

and similarly for removing a fermion.

These operations are parity preserving, and can thus be mapped to local qubit operations.

In addition, the ancillary mode is otherwise never coupled to the system, such that it does not affect its dynamics. It can be thought of as the entry point of a reservoir were fermions created (resp. annihilated) come from (resp. go away to).

Formally, the operation on $d$ is exactly the effect of one of the two Majorana operators:

$$\gamma = d^\dagger + d$$

$$\bar{\gamma} = i(d^\dagger - d)$$

and in the following we will only use the first.

As a result, in one or more embodiments, the annihilation operators $a^\dagger$ may be replaced by $a^\dagger \gamma$, and the creation operator $a$ may be replaced by $a\gamma$. The $\gamma$ cancel each other in parity-preserving terms, and a single $\gamma$ remains in parity-changing terms.

Applying this idea to the jump operators above lead to new Kraus operators: $K_0$ and $K_1 \gamma$. We used $\gamma = \gamma^\dagger$ and $\gamma^2 = 1$ to see that $K_0$ is unchanged. $K_1$ is changed only by a unitary operator, so that the probabilities of the measurement are not affected.

It can be shown that the dynamics of the system is unaffected by this addition: For clarity, we consider first the case of a single jump operator. We denote $\tilde{\rho}$ the density matrix of the system plus parity ancilla mode, and $\tilde{\mathcal{D}}$ the dissipation superoperator with the modified jump operator. The Trotterized evolution reads:

$$\tilde{\rho}(t) = e^{dt\mathcal{U}} \circ e^{dt\tilde{\mathcal{D}}} \circ e^{dt\mathcal{U}} \circ e^{dt\tilde{\mathcal{D}}} \circ \ldots \circ e^{dt\mathcal{U}} \circ e^{dt\tilde{\mathcal{D}}}(\tilde{\rho}_0) + \mathcal{O}(dt^2)$$

$$e^{dt\tilde{\mathcal{D}}}(\rho) = K_0 \rho K_0^\dagger + K_1 \gamma \rho \gamma^\dagger K_1^\dagger + \mathcal{O}(dt^2)$$

$$e^{dt\mathcal{U}}(\rho) = \mathrm{e}^{-idtH} \rho \mathrm{e}^{idtH} + \mathcal{O}(dt^2)$$

so that, developing over trajectories:

$$\tilde{\rho}(t) = \sum_{i_1,\ldots,i_N} \gamma^{|i|} e^{dt\mathcal{U}} \circ \mathcal{C}_{i_1} \circ e^{dt\mathcal{U}} \circ \mathcal{C}_{i_2} \circ \ldots \circ e^{dt\mathcal{U}} \circ \mathcal{C}_{i_N}(\tilde{\rho}_0) \gamma^{|i|} + \mathcal{O}(dt^2)$$

$$\mathcal{C}_i(\rho) = K_i \rho K_i^\dagger$$

$$|i| = \sum_{k=1}^{N} i_k$$

we used the fact that $\gamma$ commutes with $H$ and $K_0$ and anticommutes with $K_1$. Assuming the initial state is separable between the system and ancilla, i.e. $\tilde{\rho}_0 = \rho_0 \otimes \sigma_0$, and observing that $\sigma_0$ is unaffected by the Lindblad dynamics, we get:

$$\tilde{\rho}(t) = \sum_{i_1,\ldots,i_N} e^{dt\,\mathcal{U}} \circ \mathcal{C}_{i_1} \circ e^{dt\,\mathcal{U}} \circ \mathcal{C}_{i_2} \circ \ldots \circ e^{dt\,\mathcal{U}} \circ \mathcal{C}_{i_N}(\rho_0) \otimes \gamma^{|i|} \sigma_0 \gamma^{|i|} + \mathcal{O}(dt^2)$$

such that tracing over the ancilla mode yields

$$\rho(t) = e^{t\mathcal{L}}(\rho_0) + \mathcal{O}(dt^2)$$

as we wanted. One can also prove that observables and correlation functions are unaffected by the ancillary mode.

From here, the non-local quantum algorithm described above can be applied. A correct choice of a fermionic mappings will lead to a strictly local algorithm.

*Using noise:*

In the above description of the local algorithm, we mapped the original dynamics with jump operator $L = a^\dagger$ or $a$ onto one with jump operators $L_\gamma$.

Now we will need to assume a fermionic encoding has been chosen that ensures:

$$c^\dagger \gamma \equiv S_0^+ Z_0 X_1 = S_0^+ X_1$$

$$c\gamma \equiv S_0^- Z_0 X_1 = -S_0^- X_1$$

where the index 0 is for the system qubit where $L$ is applied and index 1 is for the parity ancilla qubit, where operator $\gamma$ is applied. The minus sign of the second line can be ignored, since the phase of jump operators is irrelevant. This is the case with the Jordan-Wigner encoding for instance, if the two qubits are adjacent in the encoding.

Let us assume we have perfect qubits, and one noisy qubit for each jump operator $L_k$, with an amplitude damping noise at a rate $\Gamma_k$. This physical noise may be characterized by a single jump operator $S^-$.

In one or more embodiments, a local encoding, i.e. a change of basis with local unitaries $U_\pm$ may be defined, according to which the logical jump operator $S_0^\pm X_1$ become the physical jump operator $S_0^-$:

$$S_0^- = U_\pm S_0^\pm X_1 U_\pm^\dagger$$

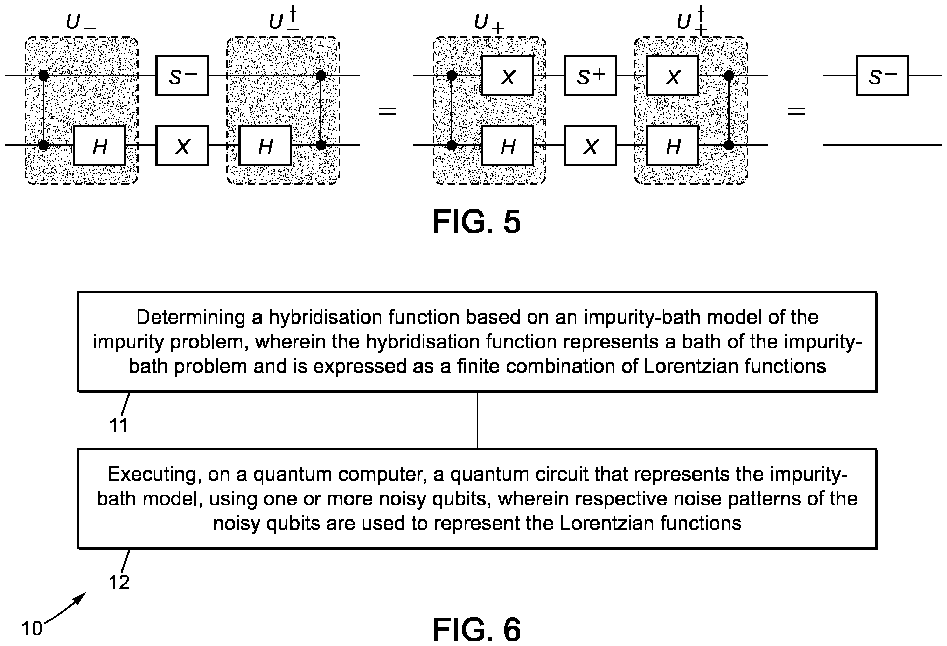

Such unitaries are shown on Fig. 5 as white boxes and black dots linked by solid lines.

Advantageously, in this encoding, the (physical) noise can be viewed as producing the (logical) dissipative effect of the used impurity-bath model.

The justification is as follows. Given any half-unitary $U$ such that $U^\dagger U = 1$, the Lindblad dynamics with Hamiltonian $H$ and jump operator $L$:

$$\frac{d\rho}{dt} = \mathcal{L}_{H,L}(\rho)$$

is equivalent to the Lindblad dynamics with Hamiltonian $\tilde{H} = UHU^\dagger$ and jump operator $\tilde{L} = ULU^\dagger$:

$$\frac{d\tilde{\rho}}{dt} = \mathcal{L}_{\tilde{H},\tilde{L}}(\tilde{\rho})$$

and $\tilde{\rho} = U\rho U^\dagger$. To recover an observable $A$ from $\tilde{\rho}$, it may be encoded as $\tilde{A} = UAU^\dagger$. In cases where the initial state, the Hamiltonian, and the measured observables are encoded with $U_\pm$, performing only the Hamiltonian evolution, e.g. by Trotterization and application of quantum gates, will reproduce the expected result. The dissipative part of the Lindbladian will be taken care of by the physical noise.

In the following, a proposed scheme according to embodiments of the present disclosure is described:

- In one or more embodiments, start with fermionic system with initial state $\rho_0$ of $n$ free fermion orbitals (= the bath,

operators $a_p^\dagger$, $a_p$), m correlated fermion orbitals (impurities), a Hamiltonian $H$ (usual Anderson impurity Hamiltonian, with a total of $n$ + m orbitals), Lindblad operators $L_p = a_p^\dagger$, $a_p$ ($k$ = 1, ... , $n$).

- In one or more embodiments, add one fermionic orbital (operators $d_p^\dagger$, $d_p$) to one or more (in some embodiments, each) of the bath orbitals (in the present subject disclosure, such an additional fermionic orbital may be indifferently referred to as a "parity orbital", an "ancillary orbital", or an "ancillary parity-tracking orbital"). No additional term is added to the Hamiltonian (we keep $H$ unchanged, only it lives in a larger Fock space so we denote it by $\tilde{H}$), but modify Lindblad operators to $\tilde{L}_p = L_p\gamma_p$ with $\gamma_p = d_p^\dagger + d_p$ (Majorana operators). The dynamics of $\tilde{\rho}$ (total of $2n$ + m orbitals) are unchanged (essentially because additional orbitals don't influence unitary dynamics, and are just used to keep track of parity). The ancillary modes initial state, denoted $\sigma_0$, is unimportant, it can be taken e.g. to all empty. The overall initial state is $\tilde{\rho}_0 = \rho_0 \otimes \sigma_0$.
- In one or more embodiments, encode this new fermionic Lindblad equation to a qubit Lindblad equation using an encoding that maps

$$\tilde{L}_p = L_p\gamma_p \leftrightarrow S_{2p}^- \otimes X_{2p+1}$$

(such a local encoding exists because the $\tilde{L}_k$ have been designed to not change the parity). This encoding gives rise to an encoded Hamiltonian $\tilde{H}_{qubit}$ and initial state $\tilde{\rho}_{0,qubit}$. The system comprises $2n$ + $m$ qubits: $n$ qubits for the bath orbitals, $n$ qubits for the parity orbitals, $m$ qubits for the impurities.
- In one or more embodiments, determine a second (qubit to qubit) encoding $U = U_1 \otimes U_2 ... \otimes U_n$ such that:

$$U_p S_{2p}^- \otimes X_{2p+1} U_p^\dagger = S_{2p}^- \otimes I_{2p+1}$$

The second encoding also transforms the Hamiltonian to $H_{qubit} = U\tilde{H}_{qubit}U^\dagger$ and the initial state $\rho_{0,qubit} = U\tilde{\rho}_{0,qubit}U^\dagger$. Namely, we have reached a qubit Lindblad equation on $2n$ + $m$ qubits, with Hamiltonian $H_{qubit}$, initial state $\rho_{0,qubit}$ and jump operators $S_{2p}^-$ acting on the "bath qubits". In some embodiments, a quantum circuit configured for performing the second encoding may be determined, and a quantum computer may be configured for executing the quantum circuit.

- In one or more embodiments, this Lindblad equation may be solved by execution on a quantum computer configured with $m$ + $n$ perfect qubits (the impurity and parity qubits) and $n$ noisy qubits (with amplitude damping noise). In some embodiments, the time evolution may be implemented by Trotterizing $H_{qubit}$, starting from the initial state $\rho_{0,qubit}$.

The proposed scheme provides the following advantages as compared to a conventional DMFT scheme which solves the AIM with noiseless fermions:

- In the conventional DMFT, we need a lot (n >> 1) of bath orbitals (i) to fit the hybridization accurately and (ii) not to see finite size effects in the dynamics and hence Green's functions. This translates, via Jordan-Wigner (or other) encodings, to a qubit computation with a large number ($m$ + $n$ >> 1) of qubits, therefore long quantum circuits, which is prone to a large number of errors if these qubits happen to be noisy.
- In contrast, the proposed scheme merely requires a few dissipative bath orbitals because (i) the fit is easier as we are fitting with Lorentzians instead of Dirac delta functions, and (ii) with only a few dissipative bath orbitals we can dampen the finite size effects. This translates in some embodiments, via the construction above, to a qubit computation with $m$ + $n$ perfect qubits and $n$ noisy qubits. One gain is therefore that with fewer qubits ($n$ is now small as compared to the conventional case) we now should obtain very good results.

Figure 6 shows a block diagram illustrating the solving (10) of an impurity problem of a solid-state material sample according to embodiments of the present subject disclosure.

One may consider solid-state material sample (e.g. a crystal sample) modeled by a lattice model.

In one or more embodiments, a hybridisation function may be determined (11) based on an impurity-bath model of the impurity problem. In some embodiments, the determined hybridisation function may represent a bath of the impurity-bath problem, and in some embodiments may further be expressed as a finite combination of Lorentzian functions.

In one or more embodiments, a quantum circuit that represents the impurity-bath model may be executed (12) on a quantum computer, using one or more noisy qubits. In some embodiments, respective noise patterns of the noisy qubits may be used to represent the Lorentzian functions.

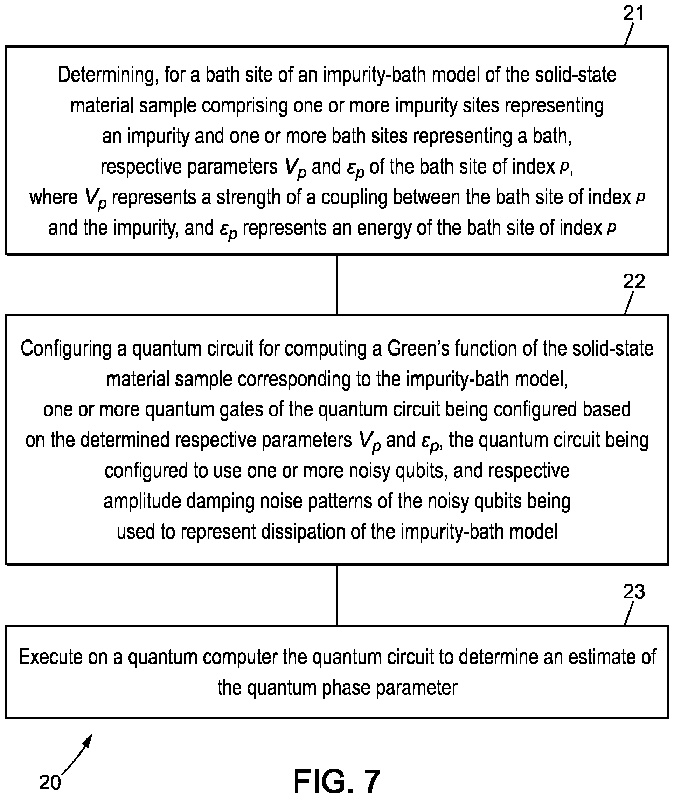

Figure 7 shows a block diagram illustrating a proposed scheme (20) for determining a quantum phase parameter characterizing a property of a quantum phase of a solid-state material sample according to embodiments of the present subject disclosure.

One may consider a solid-state sample (e.g. a crystal sample), for which a quantum phase parameter characterizing a property of a quantum phase is to be determined. One may further consider an impurity-bath model of the solid-state material sample that comprises one or more bath sites.

In one or more embodiments, for a (in some embodiments each) bath site of the impurity-bath model of the solid-state sample, respective (estimates of) parameters $V_p$ and $\varepsilon_p$ of the bath site, where $V_p$ represents a strength of the bath site, and $\varepsilon_p$ represents an energy of the bath site, may be determined (21). In the present subject disclosure, the determination of the (estimates of) parameters $V_p$ and $\varepsilon_p$ of the bath site may sometimes be referred to as the "fitting" procedure or the "fit" procedure as a reference to a fitting operation of the DMFT hybridization function in embodiments in which a DMFT procedure is used.

In one or more embodiments, a quantum circuit for computing a Green's function of the solid-state material sample corresponding to the impurity-bath model may be configured (21). In some embodiments, one or more quantum gates of the quantum circuit may be configured based on the determined respective parameters $V_p$ and $\varepsilon_p$. In some embodiments, the quantum circuit may be configured to use one or more noisy qubits, so as to advantageously harvest (use) the amplitude damping noise present in the noisy qubit(s) of the quantum circuit. In some embodiments, respective noise patterns of the noisy qubits may be used to represent an (amplitude damping) noise of the impurity-bath model.

In one or more embodiments, the quantum circuit may be executed (22) on a quantum computer to determine an estimate of the quantum phase parameter. In some embodiments, the determination of the quantum phase parameter of the solid state sample may use the execution of the determined quantum circuit on a noisy quantum computer.

In one or more embodiments, the quantum circuit may be configured to operate on a number of qubits that corresponds to a number of sites of the bath of the impurity-bath problem.

In one or more embodiments, one or more noisy qubits may be generated by adding noise to one or more respective non-noisy qubits. In some embodiments, the generated noisy qubits may be used for executing the quantum circuit.

The proposed scheme may advantageously be used to compute on a quantum computer a parameter (data representing a parameter) of the solid-state material under study. For example, embodiments of the proposed scheme may be used to compute on a quantum computer a parameter related to electrical conductivity of the solid-state material.

Various properties of a solid-state material may therefore be determined or investigated using embodiments of a proposed scheme according to the present subject disclosure.

Based on these properties, objects using the solid-state material may be produced/manufactured in order to exploit these properties.

Therefore the present subject disclosure may lead to producing or manufacturing products or objects that use a solid-state material that has been investigated using embodiments of the present subject disclosure.

In one or more embodiments, embodiments of the present subject disclosure may be used to design a solid-state material, based on properties of such material that may be determined using a proposed scheme according to the present subject disclosure. In some embodiments, the designed solid-state material may be produced / manufactured, so that a practical use of the solid-state material may be carried out.

Advantageously, some computations of proposed schemes according to the present subject disclosure may be carried out on a quantum computer, so that the proposed schemes involve a specific use of quantum processor.

In the following, embodiments of a proposed scheme according to the present subject disclosure are described.

One considers a solid-state material, such as for example a crystal material, and a model (e.g. an impurity-bath model) of such material that uses a crystal structure parameter denoted $\varepsilon(k)$, and an electronic interactions parameter denoted $U$.

In some embodiments, the crystal structure parameter $\varepsilon(k)$, and the electronic interactions parameter $U$ may be provided as input to the proposed scheme.

Given the two input parameters $\varepsilon(k)$ and $U$ related to the solid-state material, the following proposed scheme may advantageously be used to determine properties (such as electrical conductivity properties) of the solid-state material.

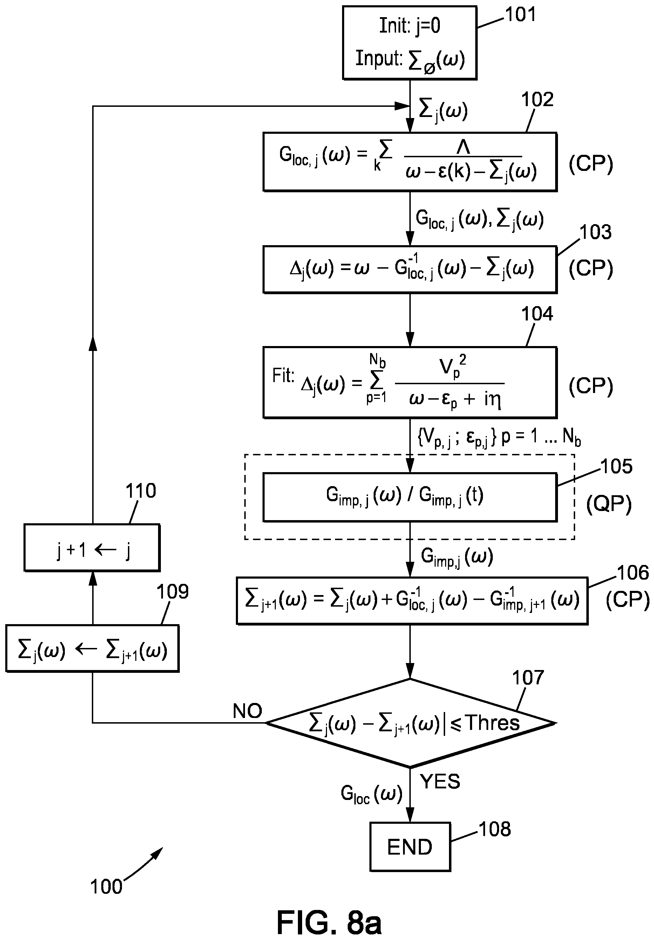

In one or more embodiments, the proposed scheme may comprise performing iterations of a loop, as illustrated by Fig. 8a.

Fig. 8a is a diagram that illustrates an iterative process for computing the (local) Green's function of a solid state material, denoted $G_{loc}(\omega)$, according to embodiments of the present subject disclosure.

As shown in Fig. 8a, in some embodiments, the iterative process (100) may be initialized with a predetermined value for the parameter $\Sigma(\omega)$ (such as for example the value 0). In some embodiments, one or more iterations of the loop may comprise one or more of the following operations, which are described in the following for an iteration of index $j$:

In some embodiments, the iteration of index $j$ may comprise: computing (102), based on the parameter $\Sigma_j(\omega)$ and the parameter $\varepsilon(k)$ (estimates of) values of a (local) Green's function of the solid state material expressed as the following complex variable function:

$$G_{loc,j}(\omega) = \sum_{k} \frac{1}{\omega - \epsilon(k) - \Sigma_j(\omega)}$$

In embodiments, the computed values of $G_{loc,j}(\omega)$ may respectively correspond to computing $G_{loc,j}(\omega)$ for a plurality of respective frequency (in some embodiments pulsation) values of $\omega$.

The sum index $k$ may belong to a so-called reciprocal space corresponding to a set of points on a space grid.

As described above, the parameter $\Sigma_j(\omega)$ may have been initialized for the first iteration of the loop at a predefined value, such as for example 0.

The local Green's function $G_{loc,j}(\omega)$ may correspond to a finite sum of Lorentzian functions.

As indicated in Fig. 8a, in some embodiments, the computation (102) may be performed on a classical (non-quantum) processor, as such computation is not computationally expensive.

In one or more embodiments, the iteration of index $j$ may further comprise: based on the values of the Green's function of the solid state material computed for the iteration $j$ ($G_{loc,j}(\omega)$) and the values of $\Sigma_j(\omega)$, computing (103) values of a hybridization function based on the following hybridization function:

$$\Delta_j(\omega) = \omega - G_{loc,j}^{-1}(\omega) - \Sigma_j(\omega)$$

In embodiments, the computed values of $\Delta_j(\omega)$ may respectively correspond to computing $\Delta_j(\omega)$ for a plurality of respective frequency (in some embodiments pulsation) values of $\omega$.

As indicated in Fig. 8a, in some embodiments, the computation (103) may be performed on a classical processor, as such computation is not computationally expensive.

In one or more embodiments, the iteration of index $j$ may further comprise: performing (104) a fitting operation to fit the hybridization function $\Delta_j(\omega)$ with the following sum of Lorentzian functions, in order to determine a set of bath parameters $\varepsilon_{p,j}$ and $V_{p,j}$:

$$\sum_{p \in \{1,\dots,n\}} \frac{V_p^2}{\omega - \epsilon_p + i\Lambda}$$

where $\varepsilon_p$ and $V_p$ are bath parameters of the used impurity-bath model, and $\Lambda$ is positive real number.

This may lead to compute values of a hybridization function expressed as a finite sum of Lorentzian functions:

$$\Delta(\omega) = \sum_{p \in \{1,\dots,n\}} \frac{V_p^2}{\omega - \epsilon_p + i\Lambda}$$

As indicated in Fig. 8a, in some embodiments, the computation (104) may be performed on a classical processor, as such computation is not computationally expensive.

In one or more embodiments, the iteration of index $j$ may further comprise: computing (105), based on the set of bath parameters $\varepsilon_{p,j}$ and $V_{p,j}$, (estimates of) values of an impurity Green's function $G_{imp,j}(\omega)$ (of an impurity-bath model of the solid state material).

In some embodiments, values of a time Green's function $G_{imp,j}(t)$ may be computed, and values of the frequency Green's function $G_{imp,j}(\omega)$ may be computed based on the values of the time Green's function $G_{imp,j}(t)$, for example using a Fourier transform of the time Green's function $G_{imp,j}(t)$.

In embodiments, the computed values of $G_{imp,j}(\omega)$ (resp. $G_{imp,j}(t)$) may respectively correspond to computing $G_{imp,j}(\omega)$ (resp. $G_{imp,j}(t)$) for a plurality of respective frequency (in some embodiments pulsation) (resp. time) values of $\omega$.

The local Green's function $G_{loc,j}(\omega)$ may correspond to a finite sum of Lorentzian functions.

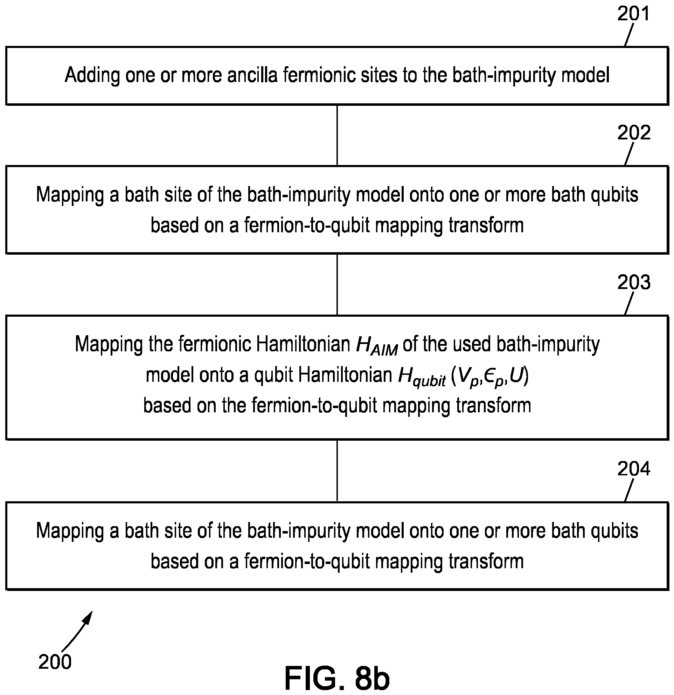

As indicated in Fig. 8a, in one or more embodiments, the computation (105) may be performed on a noisy quantum processor according to embodiments of the present subject disclosure, as described in further details in relation to Fig. 8b.

In one or more embodiments, the iteration of index $j$ may further comprise: computing (106), based on (estimates of) values of an impurity Green's function $G_{imp,j}(\omega)$ (of an impurity-bath model of the solid state material), (estimates of) values of a parameter $\Sigma_{j+1}^{\square}(\omega)$, for example using the following Dyson equation:

$$\Sigma_{j+1}^{\square}(\omega) = \Sigma_j(\omega) + G_{loc,j}^{-1}(\omega) - G_{imp,j+1}^{-1}(\omega)$$

In embodiments, the computed values of the parameter $\Sigma_{j+1}^{\square}(\omega)$ may respectively correspond to computing $\Sigma_{j+1}^{\square}(\omega)$ for a plurality of respective frequency (in some embodiments pulsation) (resp. time) values of $\omega$.

As indicated in Fig. 8a, in some embodiments, the computation (106) may be performed on a classical processor, as such computation is not computationally expensive.

Once $\Sigma_{j+1}^{\square}(\omega)$ has been computed during the current iteration (of index j), a convergence test may be performed (107) in some embodiments so as to determine whether a new iteration of the loop (iterative process) is to be performed.

For example, in some embodiments, the convergence test may comprise comparing a distance between $\Sigma_j(\omega)$ and $\Sigma_{j+1}^{\square}(\omega)$ (denoted $\left|\Sigma_j(\omega) - \Sigma_{j+1}^{\square}(\omega)\right|$ ) to a predetermined threshold *Thres,* and based on this comparison (e.g. in the case where $\left|\Sigma_j(\omega) - \Sigma_{j+1}^{\square}(\omega)\right| \leq Thres$ ) a new iteration of the loop may be performed or not.

In the case where it is determined that the loop is complete (for example, in the case where it is determined that the last computed parameter $\Sigma_{j+1}^{\square}(\omega)$ is sufficiently close to the input parameter $\Sigma_j(\omega)$ and that the current iteration of the loop has led to a parameter $\Sigma(\omega)$ that can be used to obtain a satisfactorily precise (accurate) estimate of values of a Green's function of the solid-state material $G_{loc}(\omega)$ (for example using an equation as described above for operation (102): $G_{loc,}(\omega) = \sum_k \frac{1}{\omega - \epsilon(k) - \Sigma(\omega)}$) ), the last computed values of the Green's function of the solid-state material $G_{loc,j}(\omega)$ may be used to determine various properties of the solid-state material. For example, a parameter informing on a property of the solid-state material may be determined based on the last computed values of the local Green's function $G_{loc,j}(\omega)$ (denoted $G_{loc}(\omega)$). For example, in some embodiments where the material is a crystal, values of a spectrum of the crystal may be computed as follows:

$$A_{loc}(\omega) = -\frac{1}{\pi} Im[G_{loc}(\omega)].$$

The values $A_{loc}(\omega)$ may then advantageously be used to determine properties of the crystal, e.g. as to whether the crystal is electrically conducting or not, whether the crystal is metallic or not, etc.

In the case where it is determined that the loop is not complete (for example, in the case where it is determined that the last computed parameter $\Sigma_{j+1}^{\square}(\omega)$ is not sufficiently close to the input parameter $\Sigma_j(\omega)$ and that one or more additional iterations of the loop are needed in order to obtain a parameter $\Sigma(\omega)$ that can be used to obtain a satisfactorily precise (accurate) estimate of values of a Green's function of the solid-state material $G_{loc}(\omega)$ (for example using an equation as described above for operation (102): $G_{loc}(\omega) = \sum_k \frac{1}{\omega - \epsilon(k) - \Sigma(\omega)}$)), the input parameter $\Sigma_j(\omega)$ is updated (109) with the last computed parameter $\Sigma_{j+1}^{\square}(\omega)$, and a new iteration is performed after incrementing the loop counter *j*.

In the following, embodiments of methods for producing a quantum circuit configured for harvesting the noise of qubits to obtain the impurity Green's functions according to the present subject disclosure are described. Some embodiments are directed to building (generating, producing) a quantum circuit configured for reproducing the targeted Lindbladian dynamics, before using such quantum circuit in a Green's function computing scheme.

In one or more embodiments, as described in the following, the unitary part of this dynamics is applied with quantum gates, e.g. by Trotterization of the Hamiltonian and application of the corresponding rotations. The dissipative part, on the other hand, may in some embodiments be applied passively through amplitude damping occurring on the noisy qubits all along the circuit evaluation. For this noise to be interpreted as (the correct) fermionic noise, a change of basis, or encoding may be performed in some embodiments, as described in the following.

Fig. 8b is a diagram that illustrates an exemplary scheme (200) for computing the (local) Green's function of an impurity, denoted $G_{imp}(\omega)$ or $G_{imp}(t)$, according to embodiments of the present subject disclosure.

In some embodiments, the operations shown on Fig. 8b may be performed as part of an iteration of an iterative process for computing the (local) Green's function of a solid-state material ($G_{loc}(\omega)$), according to embodiments of the present subject disclosure, such as the exemplary scheme illustrated by Fig. 8a.

One considers a solid-state material, such as for example a crystal material, and a model (e.g. a dissipative impurity-bath model) of such material that uses a crystal structure parameter denoted $\varepsilon(k)$, and an electronic interactions parameter denoted $U$.

As described in connection with Fig. 8a, bath site parameters ($\varepsilon_p$ and $V_p$) for each of the bath site of the (dissipative) impurity-bath model may have been obtained, for example by performing (104) a fitting operation to fit a hybridization function $\Delta_j(\omega)$ with a following sum of Lorentzian functions, as described in connection with Fig. 8a.

*Local noise encoding with ancilla bits*

In one or more embodiments, the dissipative impurity-bath model may be modified by adding (201) one or more ancilla fermionic (e.g. electron) sites to the model, in order to reduce the depth of the quantum circuit that is used for encoding the model.

For example, in some embodiments, for each bath site of index $p$ of one or more of the bath sites of the impurity-bath model, the corresponding jump operator (Lindblad operator) $L_p$ may be modified into $L_p\gamma_i$, where $\gamma_i$ is the Majorana operator ($\gamma_i = d_i + d_i^\dagger$) of the additional ancilla site (of index i). In some embodiments, for each bath site of index k of the bath sites of the model, the corresponding jump operator (Lindblad operator) $L_p$ may be modified into $L_p\gamma_i$, where $\gamma_i$ is the Majorana operator ($\gamma_i = d_i^{\square} + d_i^\dagger$) of the additional ancilla site (of index $i$).

This modification creates, for one or more of the bath sites of the model, pairs of sites respectively comprising a bath site of the model and the corresponding additional ancilla site. Advantageously, while not changing the dynamics of the impurity and bath, this modification allows rendering the jump operators of a bath site quadratic, which can advantageously be used to ensure that the jump operators of the bath site are local: Assuming a creation operator $a$ which adds an electron to the bath, and an annihilation operator $a^\dagger$ which removes an electron from the bath, the addition of the Majorana operator operates to move the electron, instead of creating it or removing it, thereby rendering the creation or annihilation operator local.

In order for the quantum circuit to interpret the noise of noisy qubits as a fermionic noise of a site (noise represented by the jump operators $L_p$ or $L_p\gamma_i$ depending on the embodiment), the quantum circuits may be encoded. For doing so, in some embodiments, unitary transforms (which can be represented by quantum circuits) may be determined such that a natural amplitude damping noise occurring on bath qubits may be understood (interpreted) by the quantum circuit ultimately executed as the fermion noise of interest.

*Mapping: Encoding qubit noise to fermion noise:*

In the context of the present subject disclosure which proposes to use the natural local noise of a noisy quantum computer to more efficiently compute the Green's function, the noise being deemed "local" in cases where it pertains to a single noisy qubit, the quantum circuit to be used to perform the computation may advantageously be configured to execute local jump operators in order to interpret the natural local quantum noise of the one or more processed noisy qubits as the addition or annihilation of an electron.

In one or more embodiments, one or more (in some embodiments each) bath sites of the model may be respectively mapped (202) to one or more qubits (which may be referred to in the present subject disclosure as "bath qubits"). The ancilla fermionic site that may in some embodiments be added to a bath site will also be mapped to a qubit (which may be referred to in the present subject disclosure as a "ancilla qubit" or a "parity qubit"). Therefore, in some embodiments, pairs of qubits comprising one or more bath qubits and their associated parity qubit(s) may be used, which respectively correspond to pairs of sites comprising a bath site and its associated ancilla site, as the case may be.

Using one or more additional ancilla fermionic sites may advantageously reduce the depth of the quantum subcircuits generated based thereon, as described below, as the jump operators are to be produced according to the present subject disclosure by qubit noise, which is local in quantum computers, and mapping local to non-local operators requires non-local gates. As is described below, an ordered sequence of qubits may be used in some embodiments, and the depth of the respective subcircuit for each bath site (corresponding to a processed noisy qubit) will depend on the distance in the ordered sequence of qubits between the bath qubit and the ancilla qubit.

In some embodiments, a same ancilla site may be used for several bath sites of the model, that is, may be associated with several bath sites for generating the quantum circuit.

In one or more embodiments, in order to determine a qubit Hamiltonian $\tilde{H}_{qubit}(V_p, \varepsilon_p, U)$ that corresponds to the fermionic Hamiltonian of the impurity-bath model $H_{AIM}$, a qubit Hamiltonian which describes the impurity-bath model used may be generated (203) based on the fermionic Hamiltonian of the impurity-bath model.

As discussed above, in one or more embodiments, in order for the qubit noise to be interpreted as the fermionic noise corresponding to the dynamics of the used impurity bath model, a change of basis (which may be viewed as a change of encoding) may be performed for encoding qubit noise into fermion noise.

Depending on the embodiment, any suitable fermion-to-qubit mapping transform may be used for transforming the fermionic Hamiltonian into a qubit Hamiltonian.

For example, in some embodiments, a Jordan-Wigner transform operating a fermion-to-qubit mapping may be used.

For example, in some embodiments, a fermion-to-qubit mapping may be chosen such that the fermionic jump operators of bath site $p$ be mapped onto operators of the form:

$$\xi S_{q_p}^{\pm} \prod_i P_{n_i}$$

## [Eq. 1a]

where $\xi$ is a real number, $q_p$ denotes the qubit corresponding to bath site $p$, $\{n_i\}$ is a list of qubits that does not contain $q_p$, and $P_{n_i}$ is a Pauli operator acting on qubit $n_i$.

Depending on the embodiment, such encoding (mapping) may be performed using any suitable fermion-to-qubit mapping, such as for example the Jordan-Wigner transformation, for which $\{n_i\} = \{q_p + 1, q_p + 2, ...\}$ and $P_n = Z_n$, which can be used for implementing the mapping of Eq. 1a.

Other mappings, such as for example a large class of more advanced fermion-to-qubit mappings that includes the Bravyi-Kitaev or the Verstraete-Cirac transformations, may also be used in some embodiments.

As a consequence, the impurity, the bath sites and the ancilla fermionic sites operators may be mapped onto respectively corresponding qubit operators.

In one or more embodiments, one may consider, for each bath site $p$ (i.e. each jump operator $L_p$), an encoding unitary $E_p^{\pm}$ such that

$$E_p^{\pm} S_{q_p}^{-} \left( E_p^{\pm} \right)^{\dagger} = S_{q_p}^{\pm} \prod_i P_{n_i}.$$

## [Eq. 2a]

For absorbing bath sites, the encoding circuit $E_p^{-}$ applies every $P_{n_i}$ controlled by the bath qubit $q_p$. For emitting bath sites, the encoding circuit $E_p^{+}$ is the same with an additional $X$ quantum gate applied on the bath qubit beforehand. The decoding circuit is the adjoint of the encoding circuit. Since $S^- = |0\rangle\langle 1|$, one can see that [Eq. 2a] is verified by observing that only the controls in either the encoding or decoding do fire, which makes that $\Pi_i P_{n_i}$ is applied exactly once.

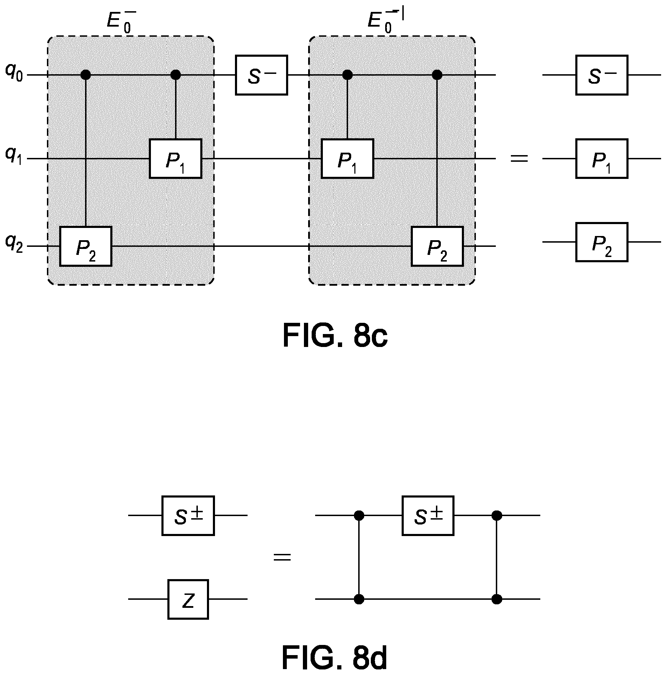

An example of quantum circuit usable for encoding qubit noise into fermion noise is given in Fig. 8c.

Fig. 8c shows an exemplary quantum circuit for encoding qubit noise into fermion noise, which may be viewed as a circuit version of Eq. 2a (jump operators encoding equation), illustrated with a 3-qubit logical jump operator on an absorbing bath site. On the left-hand-side, the physical jump operator ($S^-$) acting on a bath qubit $q_0$ is preceded by encoding $E_0^{-}$ and followed by decoding $E_0^{-\dagger}$, which results in the logical jump operator (right-hand-side). Depending on the embodiment, $P_1$ and $P_2$ may be two arbitrary Pauli operators.

Referring to Fig. 8c, amplitude damping can be interpreted as random applications of the jump operator $S^- = |0\rangle\langle 1|$. Assuming that this channel occurs between encoding and decoding, for an absorbing bath site, the qubit register starts in a superposition $\alpha|0\rangle|\phi_0\rangle + \beta|1\rangle|\phi_1\rangle$, where $|0\rangle$ and $|1\rangle$ are states of the bath qubit, and $|\phi_0\rangle$ and $|\phi_1\rangle$ are states of the rest of the register, and $|\alpha|^2 + |\beta|^2 = 1$. Encoding transforms $|\phi_1\rangle \to \Pi_i P_{n_i}|\phi_1\rangle$, but leaves the rest of the state unchanged. The jump operator $S^-$ is applied with a probability $|\beta|^2$, projecting the state onto $|0\rangle \Pi_i P_{n_i}|\phi_1\rangle$, state on which the decoding gates have no effect. Once in the state $|0\rangle$, additional $S^-$ operators cannot come (they happen with a probability zero). If no jump operator is applied, decoding gates simply undo the effect of the encoding gates. Overall, the register sees a quantum channel with a jump operator $S_{q_p}^{\pm} \prod_i P_{n_i}$, as we wanted.

In one or more embodiments, the fermion-to-qubit mapping determined for encoding qubit noise into fermion noise may be used in one or more of the two following manners.

In some embodiments, the noise encoding may be used to encode the Hamiltonian, for example by performing a change of basis such as follows:

$$H_{\mathsf{AIM}} \rightarrow \tilde{H}_{\mathsf{AIM}} = E^{\dagger} H_{\mathsf{AIM}} E$$

[Eq. 3a]

$$E = \prod_{p} E_p^{s_p}$$

[Eq. 4a]

where we defined the full encoding operator $E$, and $s_p = \pm$ depending on whether bath site $p$ is emitter (+) or absorber (-).

Note that even though the $E_p^{s_p}$ operators do not necessarily commute, the order in which they are applied is irrelevant for the resulting logical noise. In one or more embodiments, the encoding circuit E may apply them starting from the last bath site to the first in the chosen fermionic order, as it advantageously leads to simpler expressions with the Jordan-Wigner transformation.

For example, in some embodiments, the fermionic Hamiltonian $H_{AIM}$ may be mapped (e.g. using a fermion-to-qubit mapping transform) onto a qubit Hamiltonian $\tilde{H}_{qubit}(V_p, \varepsilon_p, U)$, by mapping (e.g. using a fermion-to-qubit mapping transform) the fermionic jump operators $L_p$ (in some embodiments, for pairs of bath site and ancilla site, $L_p\gamma_i$) onto qubit jump operators. In embodiments in which one or more bath sites have been paired with one or more respective ancilla fermionic sites, this mapping operation may result (through applying the mapping operation to the ancilla fermionic sites) in additional ancilla qubits, which may sometimes be referred to as parity qubits as described above.

In one or more embodiments, the mapping operation may therefore result in a set of one or more qubits comprising one or more impurity qubits respectively corresponding to the one or more impurity sites of the impurity-bath model, one or more sets of one or more bath qubits respectively corresponding to fermionic bath sites of the impurity-bath model, and in some embodiments one or more sets of one or more ancilla qubits (respectively or not, depending on the embodiment) corresponding to the one or more sets of one or more bath qubits.

Obtaining a qubit Hamiltonian that corresponds to the fermionic Hamiltonian advantageously leads to using a Hamiltonian that corresponds to the impurity-bath model and that can be described with quantum gates that operate on one or more qubits. In some embodiments, the fermionic operators of the fermionic Hamiltonian $H_{AIM}$ (depending on the embodiment, the $L_p$ or $L_p\gamma_i$ operators) may be mapped onto qubit quantum operators, based on which quantum circuits that respectively represent such qubit quantum operators may be determined.

In one or more embodiments, a quantum circuit reproducing the desired (Lindbladian) dynamics may be prepared (configured/produced) (204) based on, depending on the embodiment, one or more of i) application of the circuit for $E$, ii) application of a Trotterized evolution under the encoded hamiltonian $\tilde{H}_{AIM}$, and iii) application of the circuit for $E^{\dagger}$.

Advantageously, as discussed above, the quantum gates within each Trotter subcircuit may be kept temporally close to each other (in some embodiments as close to each other as possible). Similarly, the encoding (respectively decoding) quantum gates may be kept close to the beginning (respectively the end) of the circuit (in some embodiments as close to the beginning (respectively the end) of the circuit as possible). Indeed, noise events occurring on un-encoded bath qubits may not be interpreted as the correct fermionic noise event. Such events may produce actual errors. As a result, in one or more embodiments, time during which qubits are encoded may preferably be maximized.

For example, assuming the impurity qubits have been placed first in the fermionic order (qubit indices $\leq n_{\mathsf{imp}}$), and that the fermion-to-qubit mapping uses the Jordan-Wigner transformation, with which jump operator $p$ is proportional to

$$S_{q_p}^{\pm} \prod_{k > q_p} Z_k.$$

[Eq. 5a]

In such exemplary case, $E^{\dagger}$ is the application of a CZ gate on every pair of bath qubits, followed by $X$ gates on emitter bath qubits. The annihilation operator of bath mode $p$ may then be encoded into

$$\tilde{c}_p = E^\dagger \, S_{q_p}^- \prod_{k>q_p} Z_k \quad E = (-1)^{n_p^{\mathrm{emit}}} S_{q_p}^\pm \prod_{n_{\mathrm{imp}}<k<q_p} Z_k$$

[Eq. 6a]

with $\pm = +$ if the bath qubit $i$ is emitting, and $-$ otherwise, and $n_i^{\mathrm{emit}}$ is the number of emitter bath sites strictly after site $i$. The encoded annihilation operator of an impurity qubit at index $i$ is the same as resulting from Jordan-Wigner, but multiplied by a factor $(-1)^{n_i^{\mathrm{emit}}}$. The resulting Hamiltonian is changed only in its impurity-bath terms, which become, for an impurity site $i$ and a bath site $p$

$$\tilde{c}_i^\dagger \tilde{a}_p = (-1)^{n_i^{\mathrm{emit}}}(-1)^{n_p^{\mathrm{emit}}} S_{q_i}^+ \left( \prod_{q_i<k<n_{\mathrm{imp}}} Z_k \right) S_{q_p}^\pm \left( \prod_{k>q_p} Z_k \right)$$

[Eq. 7a]

One can see for this example that the noise encoding produces chains of $Z$ that span all qubits after $q_p$, very much like the Jordan-Wigner encoded annihilation or creation operator. This reflects the fact that the determined noise encoding is non-local. Indeed, within most fermion-to-qubit mappings, creation and annihilation operators are non-local. The $E$ operator thus maps one qubit noise onto many-qubits noise, and this necessarily causes the encoded hamiltonian $\tilde{H}_{\mathrm{AIM}}$ to have some many-qubit terms.

In the following, a description of a scheme for obtaining a noise encoding which is local by adding ancilla fermionic modes according to one or more embodiments is provided.

*Local noise encoding with ancilla qubits*

In one or more embodiments, the following operations may be performed prior to applying the fermion-to-qubit mapping in order to perform a local noise encoding operation. The above-described noise encoding may be considered non local to the extent that the fermionic jump operators add or remove an odd number of particles, i.e. they may be viewed as parity reversing as they anticommute with the parity operator $(-1)^N$ with $N$ the total number of particles operators. While there exists different fermion-to-qubit mappings (transforms) that operate to map any parity conserving operators to operators acting on a bounded number of qubits, such transforms may also map parity reversing operators to non-local qubit operators. As a consequence, in one or more embodiments, the Lindbladian is reframed in terms of parity conserving operators only prior to applying the fermion-to-qubit mapping.

As described above, in one or more embodiments, the Lindbladian may be reframed by adding one or several ancilla modes, whose Majorana operators are denoted $\gamma_\alpha$, with $\alpha$ the mode index. For example, one or more (for example each) of the bath modes $p$ may be associated with an ancilla mode $\alpha_p$ (depending on the embodiment, several bath modes may be associated to the same ancilla), and its jump operator $L_p$ may be correspondingly changed to

$$L_p \rightarrow L_p \gamma_{\alpha_p}$$

[Eq. 8a]

Adding one or several ancilla modes does not affect the dynamics of the system (impurity and bath sites), since the ancilla modes are absent from the Hamiltonian. Advantageously, it makes the jump operators two-fermion operators, and thus parity-conserving. Every time a fermion joins or leaves the system, an ancilla mode flips its occupation, such that the only operations performed by jump operators are: moving a fermion from bath to ancilla or vice-versa, and annihilating or creating a *pair* of fermions, or a superposition of these.

In one or more embodiments, the encoding operators $E_p^\pm$ may be updated with these new jump operators, and the above-described schemes for mapping the fermionic Hamiltonian $H_{\mathrm{AIM}}$ of the used impurity-bath model onto a qubit Hamiltonian $\tilde{H}_{\mathrm{qubit}}(V_p, \varepsilon_p, U)$ and for determining a quantum circuit that operate on noisy qubits and reproduces the dynamics of the impurity-bath model may be used. In embodiments in which we encode noise occurring on bath qubits only, ancilla qubits that are used may advantageously be noiseless.

As an example, if one considers a case with $N_{\mathrm{anc}}$ ancilla modes and $N_{\mathrm{bath}} = KN_{\mathrm{anc}}$ bath modes, for some integer $K$.

Using the Jordan-Wigner transform, it is preferable to keep ancilla modes close to their bath mode in the fermionic order. We thus make $N_{anc}$ blocks of $K$ consecutive bath modes and associate them to one ancilla mode, placed right after them in the fermionic order. For each bath mode $p$, $q_p$ denotes the qubit corresponding to the bath mode, and $r_p$ is the qubit of its ancilla. After Jordan-Wigner, the jump operator of bath site $p$ is proportional to

$$S_{q_p}^{\pm} \left( \prod_{q_p < k < a_p} Z_k \right) X_{r_p}$$

[Eq. 9a]

The corresponding encoding circuit $E_p^{\pm}$ is made of CZ gates between $q_p$ and each $k$ strictly between $q_p$ and $r_p$, and a CNOT gate applied on $r_p$ with $q_p$ as the control qubit. An $X$ gate follows on $q_p$ if the bath site is emitting. Each encoding circuit $E_p^{\pm}$ therefore acts on at most $K + 1$ qubits in the vicinity of $q_p$, whereas they were unbounded in absence of ancilla. In addition, the full encoding circuit E is made of $N_{bath}(K + 1)/2$ two-qubit gates, growing linearly with the bath size at fixed $K$, while it was made of $N_{bath}(N_{bath} - 1)/2$ two-qubit gates in the absence of ancilla. This confirms the local nature of the encoding with ancilla, provided the number of ancilla modes is proportional to the number of bath modes, and placed regularly in the fermionic order.

It can be verified that this encoding ensures

$$E_p^{\pm} \, S_{q_p}^{-} \, E_p^{\pm\dagger} = S_{q_p}^{\pm} \left( \prod_{q_p < k < r_p} Z_k \right) X_{r_p}$$

[Eq. 10a]

as expected. The annihilation operator on bath mode $p$ is encoded into

$$\tilde{a}_p = (-1)^{n_p^{emit}+1} S_{q_p}^{-} \left( \prod_{q_p < k < r_p} Z_k \right) X_{r_p} \left( \prod_{\substack{a\ \text{ancilla} \\ a > r_p}} Z_a \right)$$

[Eq. 11a]

and the annihilation operator on the impurity mode i is encoded into

$$\tilde{c}_i = (-1)^{n_i^{emit}} S_{q_i}^{-} \left( \prod_{q_i < k < n_{imp}} Z_k \right) \left( \prod_{a\ \text{ancilla}} Z_a \right)$$

[Eq. 12a]

The coupling term between impurity mode $i$ and bath mode $p$ is then

$$\tilde{c}_i^\dagger \tilde{a}_p = (-1)^{n_i^{\text{emit}}}(-1)^{n_p^{\text{emit}}+1}\tilde{S}_i^{\text{imp}+}\left(\prod_{\substack{a\ \text{ancilla}\\a<r_p}} Z_a\right)\tilde{S}_p^{\text{bath}-}$$

with $\qquad \tilde{S}_i^{\text{imp}+} = S_{q_i}^+\left(\prod_{q_i<k<n_{\text{imp}}} Z_k\right)$

and $\qquad \tilde{S}_p^{\text{bath}-} = S_{q_p}^-\left(\prod_{q_p<k<r_p} Z_k\right) Z_{r_p}X_{r_p}$

[Eqs. 13a, 14a, 15a]

This term applies a chain of $Z$ operators on qubits between the impurity mode i and the ancilla mode of bath $p$, but excluding every bath mode that are outside the block where $p$ lies. In the worst case it acts on $\sim N_{\text{anc}} + K = N_{\text{bath}}/K + K$ qubits.

Adding ancilla modes advantageously allows reducing the overall depth of the quantum circuit, by reducing the number of gates of the encoding circuit and by reducing the support of encoded Hamiltonian terms. The price to pay is twofold. Not only adding ancilla modes increases the total number of noiseless qubits required, but it also adds to the support of encoded Hamiltonian terms and encoding circuit. Depending on the embodiment, the overall depth of the circuit may be chosen by reaching a compromise between a tightly local encoding that requires many ancillas (small $K$), and a more loosely local encoding with a few ancillas (large $K$). Ultimately, available resources and hardware quality may dictate the best tradeoff between the number of noiseless qubits and the overall depth of the quantum circuit.

In one or more embodiments, the used fermion-to-qubit mapping may be based on a preconfigured ordering of the fermionic sites (which ordering or the sequence resulting thereof may be referred herein to as the "fermionic ordering"), in order to account for the antisymmetric nature of fermionic wavefunctions.

In one or more embodiments, prior to performing the mapping of the fermionic Hamiltonian into the qubit Hamiltonian, an ordering of the fermionic sites may be performed to generate an ordered sequence of sites (a fermionic ordering). In some embodiments, the ordering of the sites (and the resulting fermionic ordering) may be independent from the spatial configuration of the sites in the used impurity-bath model. However, the ordering of the sites may affect the depth of the quantum circuit that is ultimately generated according to the present subject disclosure, and may affect its computational performances (e.g. in terms of its computational complexity) accordingly.

In this regard, in some embodiments, the ordering of the sites may be performed using an ordering criterion. In some embodiments, in order to advantageously restraining (reducing) the depth of the quantum circuit that is ultimately generated, an ordering criterion according to which fermionic sites that (often) interact with each other may be kept close to each other in the fermionic ordering. For example, in some embodiments, an ordering criterion according to which bath sites are kept close (in the ordered sequence of sites (fermionic ordering)) to their corresponding ancilla sites (for those bath sites that are paired with a respective ancilla site), may be used. As another example, in some embodiments where the used impurity-bath model includes several impurity sites, an ordering criterion according to which impurity sites are kept close (in the ordered sequence of sites (fermionic ordering)) to each other, may be used

For example, in some embodiments, the Jordan-Wigner transform may be used to transform the $c$ and $c^\dagger$ fermionic operators into sum of Pauli strings with respective chains of $Z$ quantum gates. The above-described operation of adding one or more ancilla fermionic sites that respectively correspond to bath sites of the used impurity-bath model may advantageously be performed so as to avoid that such chains be long (resulting in a large depth of the ultimately generated quantum circuit, and therefore a large computational complexity).

In one or more embodiments, the ordering of the fermionic sites prior to the mapping operation may therefore result in an ordered sequence of qubits. The ordering index of a qubit in the qubit sequence may correspond to that of a fermionic site in the fermionic ordering.

In one or more embodiments, one or more quantum circuits (which may sometimes be referred to herein as "subcircuits") that correspond to the qubit Hamiltonian and the jump operators may be encoded (determined).

In one or more embodiments, for each pair of qubits (bath qubit and corresponding ancilla qubit), a quantum circuit is determined, which can be executed on the two qubits of the pair so as to encode the two qubits (that is, transform the noise of these two qubits). The quantum circuit corresponding to the two qubits of a pair may be configured such that, when executed on the two qubits, and waiting so that the noise of the two qubits is produced, the adjunct quantum circuit may be executed for obtaining the fermionic noise of interest.

*Quantum circuit for impurities with star geometry*

For impurities in the star geometry, as those constructed in the above-described fitting procedure according to some embodiments, the quantum circuits resulting from the above-described Trotterization have particular simplifications that allow to alleviate the effect of the chain of Z operators on ancillas from [Eq. 13a].

Using the quantum circuit simplification illustrated by Fig. 8d, the Trotter operation corresponding to the coupling term between impurity mode i and bath mode *p* may in some embodiments be written as:

$$T_{i,p} = \exp\left(-i v_{ip}\left(\tilde{c}_i^\dagger \tilde{a}_p + \text{h.c.}\right)\varDelta t\right)$$
$$= C_{i,p}\exp\left(-i\tilde{v}_{ip}\left(\tilde{S}_i^{\text{imp}+} \tilde{S}_p^{\text{bath}-} + \text{h.c.}\right)\varDelta t\right)C_{i,p}$$

[Eq. 16a]

$$\text{with } \tilde{v}_{ip} = (-1)^{n_i^{\text{emit}}}(-1)^{n_p^{\text{emit}}+1}v_{ip}, \text{ and}$$

$$C_{i,p} = \prod_{\substack{a \text{ ancilla} \\ a < r_p}} \text{CZ}_{q_i,a}$$

[Eq. 17a]

where $\text{CZ}_{q_i,a}$ is the controlled-Z operator applied between qubits $q_i$ and $a$.

Therefore, in one or more embodiments, applying the impurity-bath Trotter operators in an order where bath site is incremented before impurity site, simplifications such as

$$C_{i,p}C_{i,p+1} = \begin{cases} 1 & \text{if } p \text{ and } p+1 \text{ are in the same block} \\ \text{CZ}_{q_i,r_p} & \text{otherwise} \end{cases}$$

[Eq. 18a]

can advantageously be used.

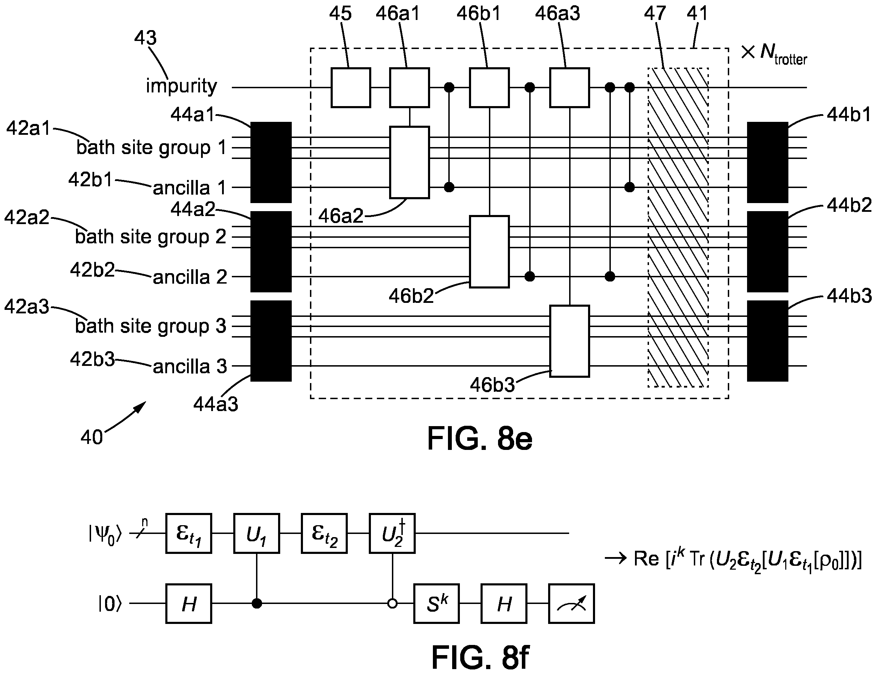

Fig. 8e illustrates an exemplary quantum circuit that illustrates this procedure in the exemplary case of a single impurity qubit coupled to nine bath qubits corresponding to an impurity-bath model with one impurity and three bath sites.

Shown on Fig. 8e is an example of quantum circuit (40) configured for harvesting amplitude damping to reproduce the dynamics (of a Lindblad equation) of an exemplary impurity-bath model according to one or more embodiments of the present subject disclosure. The exemplary impurity-bath model to which the quantum circuit (40) of Fig. 8e corresponds comprises 9 fermionic bath sites (grouped by 3 bath sites), and one impurity site. In the example shown on Fig. 8e, a single impurity qubit (43) is coupled to nine bath qubits (42a1, 42a2, 42a3), which are encoded (44a1, 44a2, 44a3, 44b1, 44b2, 44b3) to transform their natural amplitude damping into a fermionic dissipation process described by the jump operators of the above jump operators encoding equation (Eq. 7_1 and 7_2). In the example illustrated by Fig. 8e, the single impurity qubit (43) corresponds to the sole impurity site of the exemplary impurity-bath model, while each bath qubit (42a1, 42a2, 42a3) respectively correspond to one of the 9 fermionic bath sites of the exemplary impurity-bath model.

In the exemplary quantum circuit (40) shown on Fig. 8e, three ancilla qubits (42b1, 42b2, 42b3), respectively associated to the three sets of three bath qubits (42a1, 42a2, 42a3) are introduced to make the encoding (of the bath qubits) local as described above in some embodiments. In the exemplary quantum circuit (40) shown on Fig. 8e, each ancilla qubit is used to encode the group of bath qubits it is associated to (ancilla 1 for bath 1, ancilla 2 for bath 2, ancilla 3 for bath 3). The exemplary quantum circuit of Fig. 8e operates on 4 non-noisy qubit and 9 noisy qubits. One non-noisy qubit (43) is for the impurity, and 3 non-noisy qubits (42b1, 42b2, 42b3) are for the ancilla fermionic sites. The 9 noisy qubits (42a1, 42a2, 42a3) are for the bath sites.

In the exemplary quantum circuit (40) shown on Fig. 8e, the encoded Hamiltonian is applied by Trotter operations (41) using a Trotterization procedure, with the impurity Hamiltonian (45), and the coupling to each group of bath sites ((46a1, 46a2) for the bath group 1 (3 bath qubits 42a1 and 1 ancilla qubit 42b1), (46b1, 46b2) for the bath group 2 (3 bath qubits 42a2 and 1 ancilla qubit 42b2), (46a3, 46b3) for the bath group 3 (3 bath qubits 42a3 and 1 ancilla qubit 42b3)). The coupling to each group of bath sites acts on a bounded number of qubits, thanks to the local nature of the encoding, and the above-described simplification applicable to quantum circuits for impurities with star geometry, visualized by CZ gates.

*Waiting time*

In one or more embodiments, a waiting period may be enforced after each Trotter operation (ref. 47 on Fig. 8e), in order to produce the desired dissipation strength, as described in the following in further details.

As described above, in one or more embodiments, a quantum circuit that reproduces the targeted dynamics may be determined and produced based on (using) amplitude damped bath qubits. In some embodiments, the logical dissipation rate $\Lambda$ may be imposed by one or more of the physical amplitude damping characteristic time $T_1$, the physical runtime of a Trotter operation $T_{\text{trot}}$, and the logical Trotter time operation $\Delta t$. Indeed, taking a quantum trajectory interpretation of the Lindblad equation, there must be an equality between the number of physical and logical jump events in order to produce the correct dissipation strength:

$$\frac{T_{\text{trot}}}{T_1} = \Lambda \Delta t.$$

This means the amount of noise during a Trotter operation may advantageously be adjusted to obtain the wanted value for $\Lambda$.

Depending on the embodiment, adjusting the amount of noise during a Trotter operation may be performed in various ways, such as for example: i) the logical Trotter step $\Delta t$ may be tuned, keeping in mind it affects the overall bias of the computation. ii) the qubit's $T_1$ may be tuned, although this may be experimentally difficult, and iii) the physical runtime may be tuned.

In some embodiments, adjusting the amount of noise during a Trotter operation by tuning the physical runtime may be implemented by adding a moment of idling for bath qubits after each Trotter operation (as illustrated by the zebra area in Fig. 8e), i.e. a waiting time $T_{\text{wait}}$ that may be defined as follows:

$$\frac{T_{\text{trot}} + T_{\text{wait}}}{T_1} = \Lambda \Delta t.$$

Adding a moment of idling for bath qubits after each Trotter operation may advantageously be used in some embodiments to increase the dissipation rate above its minimal value $T_{\text{trot}}/T_1 \Delta t$. In some embodiments, in order to decrease the dissipation rate below its minimal value $T_{\text{trot}}/T_1 \Delta t$, $T_{\text{trot}}$ may advantageously be reduced by one or more of better optimizing the circuit, use i) (tuning the logical Trotter operation) and ii) (tuning the qubit's $T_1$) described above.

*Application to Green's function computation*

As described above, in one or more embodiments, a quantum circuit may be built (generated, determined) and produced that reproduces the dynamics of an impurity-bath model. In one or more embodiments, a Green's function of the material sample may be obtained from such a quantum circuit using any suitable method.

For example, in one or more embodiments, a method for obtaining a Green's function of the material sample based on the quantum circuit that reproduces the dynamics of an impurity-bath model, which may be based on the Hadamard test, may be used.

Further details on this method are provided below in the full generality of open quantum systems:
In an open system submitted to an evolution super-operator $\varepsilon_t$, the correlation function between two fermionic operators (that is, fermion parity reversing operators) may in some embodiments be defined as:

$$\langle \tilde{A}(t)\tilde{B}(t') \rangle = \begin{cases} Tr\big(A(-1)^{N_s}\varepsilon_{t-t'}[(-1)^{N_s}B\varepsilon_{t'}[\rho_0]]\big) & \text{if} \quad t \geq t' \\ Tr(B(-1)^{N_s}\varepsilon_{t'-t}[(-1)^{N_s}\varepsilon_t[\rho_0]A]) & \text{if} \quad t < t'. \end{cases}$$

with $N_s$ the operator giving the number of particles (of the same species as $A$ and $B$) in the system. $\tilde{A}$ and $\tilde{B}$ are the operators extended to the system and environment. For example, the initial state may be assumed to be pure: $\rho_0 = |\psi 0\rangle \langle \psi 0|$.

Using the facts that $\varepsilon_t[x]^\dagger = \varepsilon_t[x^\dagger]$, $\rho(t)(-1)^{N_s} = (-1)^{N_s}\rho(t)$ which comes from the fermionic superselection rule, and $A$ and $B$ anticommute with $(-1)^{N_s}$, this can also be written as follows:

$$\langle \tilde{A}(t)\tilde{B}(t')\rangle = \begin{cases} Tr\left(\bar{A}\ \mathcal{E}_{t-t'}[\bar{B}\ \mathcal{E}_{t'}[\rho_0]]\right) & \text{if}\quad t \geq t' \\ Tr\left(\bar{B}^{\dagger}\ \mathcal{E}_{t'-t}\left[\bar{A}^{\dagger}\ \mathcal{E}_t[\rho_0]\right]\right)^{*} & \text{if}\quad t < t'. \end{cases}$$

with $\bar{A} = A(-1)^{Ns}$ and $\bar{B} = (-1)^{Ns}B$.

Assuming a quantum circuit that produces the channel $\varepsilon_t$ for any time $t \geq 0$ is known (such as the quantum circuit that reproduces the dynamics of an impurity-bath model built and produced according to embodiments of the present subject disclosure), this correlator may in some embodiments be computed through several Hadamard tests.

Fig. 8f illustrates an exemplary quantum circuit that uses the Hadmard test to produce a correlator. In one or more embodiments, averaging measurement outcomes of the exemplary quantum circuit of Fig. 8f may be used, as such averaging measurement outcomes yields Re $[i^k Tr\,(U_2\varepsilon_{t_2}[U_1\varepsilon_{t_1}[\rho_0]])]$, where $U_1$ and $U_2$ are two unitaries and $k \in \{0,1\}$.

Running the exemplary quantum circuit of Fig. 8f for both values of k gives a way to compute $Tr\,(U_2\varepsilon_{t_2}[U_1\varepsilon_{t_1}[\rho_0]])$. By decomposing $\bar{A}$ and $\bar{B}$ into linear combinations of unitaries (it is always possible, e.g. in the Pauli basis), one can compute $\langle \tilde{A}(t)\tilde{B}(t')\rangle$ with such quantum circuits.

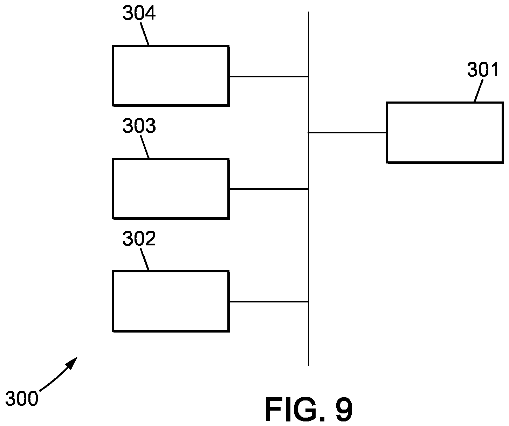

Fig. 9 illustrates an exemplary apparatus 300 configured to implement a method of determining a parameter characterizing a property of a quantum phase of a solid-state material sample in accordance with embodiments of the present subject disclosure. The apparatus 300 may, depending on the embodiment, be comprised in a quantum computing apparatus, a HPC cluster, an electronic circuit, an electronic board, an electronic component, a chip, a QPU or any other suitable processing platform.

The apparatus 300, which may comprise one or more computers, includes a control engine 301, an impurity Green's function computation engine 302, a quantum computing engine 303, a data interface engine 304, and a memory 305.

In the architecture illustrated on Fig. 9, all of the impurity Green's function computation engine 302, quantum computing engine 303, data interface engine 304, and memory 305 are operatively coupled with one another through the control engine 301.

In one or more embodiments, the impurity Green's function computation engine 302 may be configured to perform various functions or embodiments provided in the present subject disclosure, including with respect to one or more of configuring a quantum circuit for computing a Green's function of a solid-state material sample corresponding to the impurity-bath model, and executing, on a quantum computer, the quantum circuit to determine an estimate of a quantum phase parameter characterizing a property of a quantum phase of the solid-state material sample. In some embodiments, the impurity Green's function computation engine 302 may be implemented in software and incorporated in a computing machine running on a hybrid computer, configured according to embodiments of the present subject disclosure.

In one or more embodiments, the quantum computing engine 303 may be configured with one or more quantum processing resources (e.g. one or more QPUs) to perform various functions or embodiments provided in the present subject disclosure, including with respect to one or more of configuring a quantum circuit for computing a Green's function of a solid-state material sample corresponding to the impurity-bath model, and executing, on a quantum computer, the quantum circuit to determine an estimate of a quantum phase parameter characterizing a property of a quantum phase of the solid-state material sample. Further, in one or more embodiments, the quantum computing engine 303 may be further configured with one or more classical processing resources (e.g. one or more CPUs) to perform various functions or embodiments provided in the present subject disclosure, including with respect to one or more of determining, for a bath site of an impurity-bath model of the solid-state material sample, respective parameters $V_p$ and $\varepsilon_p$ of the bath site, where $V_p$ represents a strength of the bath site, and $\varepsilon_p$ represents an energy of the bath site, and configure a quantum circuit for computing a Green's function of the solid-state material sample corresponding to the impurity-bath model.

In one or more embodiments, the data interface engine 304 may be configured to provide a data interface to the apparatus 300, including for exchanging data with another apparatus or receiving, through a user input interface, input data to be used for implementing a method according to embodiments of the present subject disclosure.

The control engine 301 includes one or more of quantum and classical processors, which may be any suitable microprocessor, microcontroller, Field Programmable Gate Arrays (FPGA), Application Specific Integrated Circuits (ASIC), Digital Signal Processing chip, and/or state machine, or a combination thereof. According to various embodiments, one or more of the computers can be configured as a multi-processor computer having multiple processors for providing parallel computing. The control engine 301 may also comprise, or may be in communication with, computer storage media, such as, without limitation, the memory 305, capable of storing computer program instructions or software code that, when executed by the processor, causes the processor to perform the elements described herein. In addition, the memory 305 may be any type of data storage computer storage medium, capable of storing multimedia content data, service data, service list data, customization data, personalization data and/or channel data for use according to one or more embodiments of the present subject disclosure, coupled to the control engine 301 and operable with the data interface engine 304, the quantum computing engine 303, and the impurity Green's function computation engine 302 to facilitate processing of data stored in association therewith.

In embodiments of the present subject disclosure, the apparatus 300 is configured for performing the processing methods described herein.

It will be appreciated that the apparatus 300 shown and described with reference to Fig. 9 is provided by way of example only. Numerous other architectures, operating environments, and configurations are possible. Other embodiments of the apparatus may include fewer or greater number of components and may incorporate some or all of the functionality described with respect to the apparatus components shown in Fig. 9. Accordingly, although the control engine 301, data interface engine 304, quantum computing engine 303, impurity Green's function computation engine 302, and memory 305 are illustrated as part of the apparatus 300, no restrictions are placed on the location and control of components 101 - 105. In particular, in other embodiments, components 101 - 105 may be part of different entities or computing systems.

In one or more embodiments, a digital quantum processor (e.g. a gate-based quantum processor) may advantageously be used to solve the impurity-bath problem.

Although this subject disclosure has been disclosed in the context of certain preferred embodiments, it should be understood that certain advantages, features and aspects of the systems, devices, and methods may be realized in a variety of other embodiments. Additionally, it is contemplated that various aspects and features described herein can be practiced separately, combined together, or substituted for one another, and that a variety of combination and sub-combinations of the features and aspects can be made and still fall within the scope of the subject disclosure. Furthermore, the systems and devices described above need not include all of the modules and functions described in the preferred embodiments.

Information and signals described herein can be represented using any of a variety of different technologies and techniques. For example, data, instructions, commands, information, signals, bits, symbols, and chips can be represented by voltages, currents, electromagnetic waves, magnetic fields or particles, optical fields or particles, or any combination thereof.

Depending on the embodiment, certain acts, events, or functions of any of the methods described herein can be performed in a different sequence, may be added, merged, or left out altogether (e.g., not all described acts or events are necessary for the practice of the method). Moreover, in certain embodiments, acts or events may be performed concurrently rather than sequentially.

**Claims**

1. A computer-implemented method for determining a quantum phase parameter characterizing a property of a quantum phase of a solid-state material sample, the method comprising:

   Determining, for a bath site of an impurity-bath model of the solid-state material sample comprising one or more impurity sites representing an impurity and one or more bath sites representing a bath, respective parameters $V_p$ and $\varepsilon_p$ of the bath site of index $p$, wherein $V_p$ represents a strength of a coupling between the bath site of index p and the impurity, and $\varepsilon_p$ represents an energy of the bath site of index $p$;
   Configuring a quantum circuit for computing a Green's function of the solid-state material sample corresponding to the impurity-bath model, wherein one or more quantum gates of the quantum circuit are configured based on the determined respective parameters $V_p$ and $\varepsilon_p$, wherein the quantum circuit is configured to use one or more noisy qubits, and wherein respective amplitude damping noise patterns of the noisy qubits are used to represent dissipation of the impurity-bath model; and
   Executing, on a quantum computer, the quantum circuit to determine an estimate of the quantum phase parameter.

2. The method according to claim 1, wherein the quantum circuit is configured to operate on a number of noisy qubits that corresponds to a number of sites of the bath of the impurity-bath problem.

3. The method according to any of the previous claims, further comprising: producing the solid-state material sample for using the determined parameter characterizing the property of a quantum phase of the solid-state material sample.

4. The method according to any of the previous claims, wherein the quantum circuit is executed on the quantum computer to compute an estimate of a Green's function of the solid-state material sample, and wherein the estimate of the quantum phase parameter is determined based on the computed Green's function.

5. The method according to any of the previous claims, further comprising:

performing one or more iterations of an impurity-bath model resolution loop for a bath site of index $p$, wherein an iteration of index $j$ of the impurity-bath model resolution loop comprises:

computing (102), based on a structure parameter $\varepsilon(k)$ of the solid-state material sample, and a parameter $\Sigma_j(\omega)$, estimates of values of a local Green's function of the solid state material expressed as the following complex variable function:

$$G_{loc,j}(\omega) = \sum_k \frac{1}{\omega - \epsilon(k) - \Sigma_j(\omega)}$$

wherein the parameter $\Sigma_j(\omega)$ is set to an initial value $\Sigma_0(\omega)$ for performing the first iteration of the impurity-bath model resolution loop;

based on the estimates of values of the Green's function of the solid state material $G_{loc,j}(\omega)$ computed for the iteration $j$, and values of the parameter $\Sigma_j(\omega)$, computing (103) estimates of values of a hybridization function based on the following hybridization function:

$$\Delta_j(\omega) = \omega - G_{loc,j}^{-1}(\omega) - \Sigma_j(\omega)$$

performing (104) a fitting operation to fit the hybridization function $\Delta_j(\omega)$ with a sum of Lorentzian functions, in order to determine a set of bath parameters $\varepsilon_{p,j}$ and $V_{p,j}$, wherein the sum of Lorentzian functions is based on:

$$\sum_{p \in \{1,\dots,n\}} \frac{V_p^2}{\omega - \epsilon_p + i\Lambda}$$

where $\varepsilon_p$ and $V_p$ are the bath parameters of the impurity-bath model, and $\Lambda$ is a real positive number;

computing (105), based on the set of bath parameters $\varepsilon_{p,j}$ and $V_{p,j}$, estimates of values of an impurity Green's function $G_{imp,j}(\omega)$ corresponding the impurity-bath model of the solid state material; and

computing (106), based on the estimates of values of the impurity Green's function $G_{imp,j}(\omega)$, estimates of values of a parameter $\Sigma_{j+1}^{\square}(\omega)$.

6. The method according to claim 4, wherein the quantum circuit is configured for performing the computing (105), based on the set of bath parameters $\varepsilon_{p,j}$ and $V_{p,j}$, of the estimates of values of the impurity Green's function $G_{imp,j}(\omega)$ corresponding to the impurity-bath model of the solid state material, when executed on a noisy quantum computer.

7. The method according to any of the preceding claims, wherein the quantum circuit for computing the Green's function of the solid-state material sample is configured for performing a quantum computation with $m + l$ perfect qubits, and $n$ noisy qubits, wherein the number $m$ is a non-zero natural integer that is a number of impurity qubits corresponding to a number of sites in the impurity of the impurity-bath model, the number $l$ is a natural integer that is a number of additional qubits corresponding to a number of additional fermionic sites added to the impurity-bath model, and the number $n$ of noisy qubits is a non-zero natural integer that is a number of bath qubits corresponding to a number of bath fermionic sites of the impurity bath model.

8. The method according to any of the preceding claims, further comprising:

mapping a bath site of the impurity-bath model onto a bath qubit using a fermion-to-qubit mapping transform;

Mapping a fermionic Hamiltonian $H_{AIM}$ of the impurity-bath model onto a qubit Hamiltonian $\tilde{H}_{qubit}(V_p, \varepsilon_p, U)$ based on the fermion-to-qubit mapping transform

Wherein the quantum circuit is determined based on the qubit Hamiltonian $\tilde{H}_{qubit}(V_p, \varepsilon_p, U)$.

9. The method according to claim 8, wherein the qubit Hamiltonian $\tilde{H}_{qubit}(V_p, \varepsilon_p, U)$ is encoded into an encoded Hamiltonian, and the encoded Hamiltonian is applied using a Trotterization procedure.

10. The method according to any of the preceding claims, further comprising: adding one or more additional fermionic sites to the impurity-bath model.

11. The method according to claim 9, wherein, for a bath site of index *k* of one or more of the bath sites of the impurity-bath model, a corresponding jump operator $L_p$ is modified into $L_p \gamma_i$, wherein the operator $\gamma_i$ acts on an additional fermionic site of index i among the one or more additional fermionic sites is configured to perform the following operation:

$$\gamma_i = d_i^{\square} + d_i^{\dagger} .$$

12. A quantum computer apparatus, the apparatus comprising a quantum processor and a memory operatively coupled to the processor, wherein the apparatus is configured to implement, using the quantum processor, a method according to any of claims 1 to 11.

13. A computer program product comprising computer program code embodied in a computer readable medium, said computer program code comprising instructions to, when provided to a quantum computer system and executed, cause said computer to implement, using the quantum processor, a method according to any of claims 1 to 11.

many perfect qubits

$$-\frac{1}{\pi}\mathrm{Im}\Delta_{\mathrm{aux}}^{R}(\omega) = \sum_{p} V_{p}^{2}\,\delta(\omega-\epsilon_{p})$$

a few noisy qubits

$$-\frac{1}{\pi}\mathrm{Im}\Delta_{\mathrm{aux}}^{R}(\omega) = \sum_{p} V_{p}^{2}\,\frac{\Lambda}{(\omega-\epsilon_{p})^{2}+\Lambda^{2}}$$

FIG. 1

FIG. 2

FIG. 3

FIG. 4

EP 4 734 014 A1

FIG. 5

Determining a hybridisation function based on an impurity-bath model of the impurity problem, wherein the hybridisation function represents a bath of the impurity-bath problem and is expressed as a finite combination of Lorentzian functions

11

Executing, on a quantum computer, a quantum circuit that represents the impurity-bath model, using one or more noisy qubits, wherein respective noise patterns of the noisy qubits are used to represent the Lorentzian functions

12

10

FIG. 6

21

Determining, for a bath site of an impurity-bath model of the solid-state material sample comprising one or more impurity sites representing an impurity and one or more bath sites representing a bath, respective parameters $V_p$ and $\varepsilon_p$ of the bath site of index $p$, where $V_p$ represents a strength of a coupling between the bath site of index $p$ and the impurity, and $\varepsilon_p$ represents an energy of the bath site of index $p$

22

Configuring a quantum circuit for computing a Green's function of the solid-state material sample corresponding to the impurity-bath model, one or more quantum gates of the quantum circuit being configured based on the determined respective parameters $V_p$ and $\varepsilon_p$, the quantum circuit being configured to use one or more noisy qubits, and respective amplitude damping noise patterns of the noisy qubits being used to represent dissipation of the impurity-bath model

23

Execute on a quantum computer the quantum circuit to determine an estimate of the quantum phase parameter

20

# FIG. 7

FIG. 8a

201

Adding one or more ancilla fermionic sites to the bath-impurity model

202

Mapping a bath site of the bath-impurity model onto one or more bath qubits based on a fermion-to-qubit mapping transform

203

Mapping the fermionic Hamiltonian $H_{AIM}$ of the used bath-impurity model onto a qubit Hamiltonian $H_{qubit}$ $(V_p, \epsilon_p, U)$ based on the fermion-to-qubit mapping transform

204

Mapping a bath site of the bath-impurity model onto one or more bath qubits based on a fermion-to-qubit mapping transform

200

# FIG. 8b

FIG. 8c

FIG. 8d

FIG. 8e

$$\rightarrow \mathrm{Re}\,[i^k\,\mathrm{Tr}\,(U_2\mathcal{E}_{t_2}[U_1\mathcal{E}_{t_1}[\rho_0]])]$$

FIG. 8f

EP 4 734 014 A1

FIG. 9

Europäisches
Patentamt

European
Patent Office

Office européen
des brevets

# EUROPEAN SEARCH REPORT

**Application Number**

EP 24 30 6810

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AYRAL THOMAS ET AL: "Quantum computing with and for many-body physics", THE EUROPEAN PHYSICAL JOURNAL A ; HADRONS AND NUCLEI, vol. 59, no. 10, 27 September 2023 (2023-09-27), XP093170324, Bo ISSN: 1434-601X, DOI: 10.1140/epja/s10050-023-01141-1 Retrieved from the Internet: URL:https://arxiv.org/pdf/2303.04850> * abstract * * chapters 1 - 9; page 1 - page 40; figures 1.1 - 8.7 * ----- | 1-13 | INV. G06N10/60 G06N10/20 G16C60/00 |
| X | JUHA LEPP\"AKANGAS ET AL: "A quantum algorithm for solving open system dynamics on quantum computers using noise", ARXIV.ORG, CORNELL UNIVERSITY LIBRARY, 201 OLIN LIBRARY CORNELL UNIVERSITY ITHACA, NY 14853, 14 February 2023 (2023-02-14), XP091437282, * abstract * * chapters I - Appendix E; page 1 - page 18; figures 1-8 * ----- | 1-13 | |
| | | | **TECHNICAL FIELDS SEARCHED (IPC)** |
| | | | G06N G16C |

The present search report has been drawn up for all claims

| Place of search | Date of completion of the search | Examiner |
|---|---|---|
| The Hague | 7 April 2025 | Hasnas, Sergiu |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................................

& : member of the same patent family, corresponding document

EPO FORM 1503 03.82 (P04C01)